# EUROPÄISCHE PATENTSCHRIFT

(11) **EP 1 794 597 B1**
(45) Veröffentlichungstag und Bekanntmachung des Hinweises auf die Patenterteilung: **11.03.2009**
(21) Anmeldenummer: 05790832.9
(22) Anmeldetag: 19.09.2005
(51) Int. Cl.: G01N 35/04, G01N 3/40, G01N 33/15, B65G 47/14

(54) **VORRICHTUNG UND VERFAHREN ZUR BESTIMMUNG EINES PARAMETERS VON OBJEKTEN**
DEVICE AND METHOD FOR DETERMINING AN OBJECT PARAMETER
DISPOSITIF ET PROCEDE DE DETERMINATION D'UN PARAMETRE D'OBJETS

(30) Priorität: 20.09.2004 DE 102004045847
(43) Veröffentlichungstag der Anmeldung: 13.06.2007
(73) Patentinhaber: Kraemer, Thilo, 64291 Darmstadt (DE)
(72) Erfinder: Kraemer, Thilo, 64291 Darmstadt (DE)
(74) Vertreter: Mierswa, Klaus
(86) Internationale Anmeldenummer: PCT/DE2005/001645
(87) Internationale Veröffentlichungsnummer: WO 2006/032246

(56) Entgegenhaltungen:
- EP-A- 0 104 062
- WO-A-01/90744
- US-A- 4 236 413
- US-A- 4 660 713
- US-A- 4 884 463
- US-A1- 2003 209 098

## Beschreibung

### Technisches Gebiet:

Die Erfindung betrifft eine Vorrichtung sowie ein Verfahren zur Bestimmung wenigstens eines physikalischen oder chemischen Parameters von Objekten insbesondere von pharmazeutischen Objekten, wie Tabletten.

### Stand der Technik:

Es sind Vorrichtung zur Qualitätskontrolle fester pharmazeutischer Erzeugnisse, wie beispielsweise Tabletten, Pillen, Oblongs oder dergleichen bekannt, bei denen derartige Erzeugnisse einzeln einer oder verschiedenen Prüfeinrichtungen, beispielsweise für Gewicht, Härte, Abmessungen, Abriebeigenschaften oder dergleichen, zugeführt werden.

Die WO01/90744 offenbart eine Vorrichtung zur automatischen Qualitätskontrolle von Prüflingen, wie Tablets, Tabletten, Pillen oder Dragees, zur Bestimmung von mechanisch-physikalischen Eigenschaften derselben, wie Gewicht, Abmessungen, Zerfallszeit in einem Medium sowie Härte, bestehend aus einer Zuführeinrichtung und einem Transportstern mit peripher angeordneten Aufnahmekammern für je einen Prüfling sowie einem Härtetester, gegebenenfalls einer Waage sowie einer Einrichtung zur Bestimmung der Abmessungen des Prüflings, wobei an einen Abgang des Transportsterns zur Übernahme des Prüflings ein Linearförderer angeschlossen ist, an den sich der Härtetester anschließt.

Aus der DE 103 16 024 A1 geht eine Vorrichtung zur Qualitätskontrolle pharmazeutischer Erzeugnisse, wie Tabletten, hervor, welche eine Prüfeinrichtung mit einem Sensor zur Prüfung eines oder mehrerer Parameter der Erzeugnisse aufweist. Die Vorrichtung besteht aus einem Gehäuse mit einem darüber angeordneten, geschlossenen Prüfraum, welcher durch einen Boden und eine darauf aufgesetzte Schutzhaube gebildet ist, in welchem sich Prüfeinrichtungen befinden. Antriebe und gegebenenfalls Versorgungseinrichtungen der im Prüfraum angeordneten Prüfeinrichtungen und Transporteinrichtungen sind in dem gegenüber dem Prüfraum und der Umgebung spritzwasser- und staubdicht gekapselten separaten Gehäuse angeordnet. Wenigstens Teile der Zuleitungen zu den Antrieben und den Versorgungseinrichtungen für die Energieversorgung der Prüfeinrichtung innerhalb des Gehäuses sind in ebenfalls spritzwasser- und staubdicht gekapselter Weise nach außerhalb des geschlossenen Prüfraumes geführt.

Aus der EP 0 618 447 B1 ist ein Verfahren zum automatischen Prüfen und Qualitätsbestimmen von Tabletten oder Pillen, welche aus einer Pressmaschine kommen und in eine Mehrzahl von Behältern gefördert werden, bekannt. Die Behälter sind auf je einer Waage angeordnet. Für jeden Behälter erfolgt eine Identifizierung. Dem Tabletten- oder Pillenstrom, der in den momentan zu befüllenden Behälter läuft, werden Tabletten- oder Pillenprüflinge entnommen und geprüft. Die Prüfergebnisse werden gespeichert.

Aus der US 4,660,713 ist eine Transporteinheit für Tabletten mit einer horizontalen Führung (horizontal guiding track) bekannt, über welche die Tabletten zu Prüfeinrichtungen (tablet testing stations) befördert werden.

Die DE 42 41 985 A1 offenbart eine Vorrichtung mit einem Drehteller mit kreisrunden Aufnahmen zum Prüfen von Tabletten. Die Tabelleten werden mit dieser Vorrichtung einem Härtetest unterworfen.

Aus der US 4,884,463 ist eine Vorrichtung mit einem Sensor zur Messung der Dicke und Mitteln zur Bestimmung des Gewichts eines Artikels bekannt. Aus dem gemessenen Gewicht und einer Standard-Dicke des Artikels wird eine radiale Abmessung (radial dimension) des Artikels berechnet. Das Ergebnis der Berechnung wird dazu herangezogen, den Artikel unter den Sensor zu bewegen und dort zu zentrieren.

Aus der EP 0 104 062 A2 geht eine automatische Wiegevorrichtung zur Wägung von solchen Artikeln hervor, deren spezifisches Gewicht (specific gravity) nicht konstant ist. Diese Vorrichtung weist Mittel zur Formung (shapimg means) und Mittel zur Druckmessung (pressure sensing means) auf. In Abhängigkeit vom Ergebnis der Druckmessung wird ein innerhalb vorgegebener Grenzen liegendes Zielgewicht eingestellt.

Die US 2003/0209098 A1 betrifft eine Vorrichtung zur Ausrichtung von Tabletten auf einer Meßstation eines Tabelettentesters mit einem Verschiebetisch (transfer table), zwei parallelen Rollen (rollers) und einem Antrieb. Dieser treibt die Rollen in entgegengesetzten Richtungen an, so dass eine Test-Tablette, welche auf die Rollen aufgebracht wird, automatisch entsprechend den Achsen der Rollen ausgerichtet wird.

Aus der US 4,236,413 geht eine Vorrichtung mit einem Förderer (conveyor) und Mitteln zur Messung der Dicke und der Druckfestigkeit (compression strength) von tabelettenförmigen Probekörpern (specimen) hervor. Die Vorrichtung umfasst eine Säuberungseinrichtung, mittels welcher Reste von Probekörpern von dem Mittel zur Messung der Druckfestigkeit entfernbar sind, und ferner einen Anschlag (stop member), von welchem der Probekörper mittels des Förderers um eine kleine Strecke beabstandbar ist, nachdem der Probekörper am Anschlag angeschlagen ist.

### Technische Aufgabe:

Der Erfindung liegt die Aufgabe zu Grunde, ein gegenüber dem Stand der Technik verbessertes Verfahren und eine gegenüber dem Stand der Technik verbesserte Vorrichtung zur Bestimmung wenigstens eines physikalischen oder chemischen Parameters, wie Gewicht, Härte, Dicke, Länge, Durchmesser, Löslichkeit, Schadstoffkonzentration, von Objekten von fester oder gelartiger Konsistenz, insbesondere von pharmazeutischen Objekten, wie Tabletten, Pillen oder Oblongs anzugeben.

Die Aufgabe wird erfindungsgemäß gelöst durch eine Vorrichtung zur Bestimmung wenigstens eines physikalischen oder chemischen Parameters, wie Gewicht, Härte, Dicke, Länge, Durchmesser, Löslichkeit, Schadstoffkonzentration, von Objekten von fester oder gelartiger Konsistenz, insbesondere von pharmazeutischen Objekten, wie Tabletten, Pillen oder Oblongs, mit einer Zuführeinrichtung, einer Unterlage, einer Verschiebeeinrichtung, mindestens einer Prüfeinrichtung, wie Waage, Härteprüfer oder Längenmeßeinrichtung, sowie einem Hub- und Senkmechanismus, welcher imstande ist, den vertikalen Abstand zwischen Verschiebeeinrichtung und Unterlage zu verändern, wobei
- die Zuführeinrichtung imstande ist, in einem Schritt a) eines der Objekte auf einen vorgegebenen Startbereich der Unterlage zu werfen, fallenzulassen, aufzulegen oder in sonstiger Weise lose auf der Unterlage anzuordnen, wobei die Verschiebeeinrichtung oberhalb der Unterlage in einer Anfangsstellung angeordnet ist, in welcher der lichte vertikale Abstand zwischen Verschiebeeinrichtung und Unterlage kleiner ist als die Vertikalausdehnung des Objektes oder den Wert Null aufweist,
- die Verschiebeeinrichtung imstande ist, in einem Schritt b) mittels eines Antriebes eine Verschiebebewegung auszuführen und hierdurch dieses Objekt unter Entfernung desselben aus dem Startbereich auf der Unterlage in den Erfassungsbereich der Prüfeinrichtung zu verschieben, so dass diese imstande ist, in einem Schritt c) den oder wenigstens einen der zu bestimmenden physikalischen oder chemischen Parameter dieses Objekts zu erfassen,
- der Hub- und Senkmechanismus imstande ist, in einem Schritt d) einen lichten vertikalen Mindestabstand zwischen Unterlage und Verschiebeeinrichtung herzustellen, welcher größer ist als die Vertikalausdehnung des Objektes, wobei das Objekt auf der Unterlage verbleibt,
- die Verschiebeeinrichtung imstande ist, anschließend in einem Schritt e) mittels des Antriebes eine der Verschiebebewegung entgegengesetzt gleiche Gegenbewegung auszuführen, ohne hierbei den Mindestabstand zu unterschreiten und somit ohne das Objekt zu verschieben oder zu berühren, und
- der Hub- und Senkmechanismus imstande ist, anschließend in einem Schritt f) den lichten vertikalen Abstand zwischen Verschiebeeinrichtung und Unterlage auf den Wert Null oder auf einen Wert, welcher kleiner ist als die Vertikalausdehnung des Objektes, zu vermindern und somit die Verschiebeeinrichtung imstande ist, gegenüber der Unterlage in die Anfangsstellung zurückzukehren,
wobei der Antrieb ein Schwenkantrieb und die Verschiebebewegung sowie die Gegenbewegung jeweils Schwenkbewegungen sind.

Eine Erfindungsgemäße Vorrichtung umfasst also eine solche zur Bestimmung wenigstens eines physikalischen oder chemischen Parameters, wie Gewicht, Härte, Dicke, Länge, Durchmesser, Löslichkeit, Schadstoffkonzentration, von Objekten von fester oder gelartiger Konsistenz, insbesondere von pharmazeutischen Objekten, wie Tabletten, Pillen oder Oblongs, mit einer Zuführeinrichtung, einer Unterlage, einer Verschiebeeinrichtung, mindestens einer Prüfeinrichtung, wie Waage, Härteprüfer oder Längenmeßeinrichtung, sowie einem Hub- und Senkmechanismus, welcher imstande ist, den vertikalen Abstand zwischen Verschiebeeinrichtung und Unterlage zu verändern, wobei
- die Zuführeinrichtung in einem Schritt a) eines der Objekte auf einen vorgegebenen Startbereich der Unterlage wirft, fallenläßt, auflegt oder in sonstige Weise lose auf der Unterlage anordnet, wobei die Verschiebeeinrichtung oberhalb der Unterlage in einer Anfangsstellung angeordnet ist, in welcher der lichte vertikale Abstand zwischen Verschiebeeinrichtung und Unterlage kleiner ist als die Vertikalausdehnung des Objektes oder den Wert Null aufweist,
- die Verschiebeeinrichtung in einem Schritt b) mittels eines Antriebes eine Verschiebebewegung ausführt und hierdurch dieses Objekt unter Entfernung desselben aus dem Startbereich auf der Unterlage in den Erfassungsbereich der Prüfeinrichtung verschiebt, so dass diese imstande ist, in einem Schritt c) den oder wenigstens einen der zu bestimmenden physikalischen oder chemischen Parameter dieses Objekts zu erfassen,
- der Hub- und Senkmechanismus in einem Schritt d) einen lichten vertikalen Mindestabstand zwischen Unterlage und Verschiebeeinrichtung herstelllt, welcher größer ist als die Vertikalausdehnung des Objektes,
wobei das Objekt auf der Unterlage verbleibt,
- die Verschiebeeinrichtung anschließend in einem Schritt e) mittels des Antriebes eine der Verschiebebewegung entgegengesetzt gleiche Gegenbewegung ausführt, ohne hierbei den Mindestabstand zu unterschreiten und somit ohne das Objekt zu verschieben oder zu berühren, und
- der Hub- und Senkmechanismus anschließend in einem Schritt f) den lichten vertikalen Abstand zwischen Verschiebeeinrichtung und Unterlage auf den Wert Null oder auf einen Wert, welcher kleiner ist als die Vertikalausdehnung des Objektes, vermindert und somit die Verschiebeeinrichtung gegenüber der Unterlage in die Anfangsstellung zurückkehrt.

Gemäß einer alternativen Variante ist die erfindungsgemäße Vorrichtung so eingerichtet, dass die Verschiebeeinrichtung anstelle der Schritte e) und f) mittels des Antriebes eine der Verschiebebewegung nicht entgegengesetzt gleiche Rückkehrbewegung ausführt, während welcher die Verschiebeeinrichtung das Objekt weder verschiebt noch berührt und nach welcher sich die Verschiebeeinrichtung wieder in der Ausgangsstellung befindet.

Erfindungsgemäß ist der Antrieb ein Schwenkantrieb, wobei die Verschiebebewegung sowie die Gegenbewegung jeweils Schwenkbewegungen sind.

Bevorzugt ist die Verschiebeeinrichtung imstande,
- im Schritt b) mittels des Schwenkantriebes als Verschiebebewegung in einer vorgegebenen Drehrichtung eine Vorwärts-Schwenkung von einer Anfangs- in eine Endstellung um einen vorgegebenen Vorwärts-Schwenkwinkel ϕ um eine vertikale oder zur Unterlage senkrechte Schwenkachse auszuführen und hierdurch dieses Objekt unter Entfernung desselben aus dem Startbereich auf der Unterlage in den Erfassungsbereich der Prüfeinrichtung zu verschieben, so dass diese imstande ist, im Schritt c) den oder wenigstens einen der zu bestimmenden physikalischen oder chemischen Parameter dieses Objekts zu erfassen,
- und im Schritt e) als Gegenbewegung mittels des Schwenkantriebes entgegen der vorgegebenen Drehrichtung eine Rückwärts-Schwenkung um einen dem Vorwärts-Schwenkwinkel entgegengesetzt gleichen Rückwärts-Schwenkwinkel auszuführen, ohne hierbei den Mindestabstand zu unterschreiten und somit ohne das Objekt zu verschieben oder zu berühren.

Gemäß einer Ausführungsform ist die Verschiebeeinrichtung imstande, im Schritt e) mittels des Schwenkantriebes anstelle der Gegenbeegung bzw. der Rückwärts-Schwenkung eine weitere Vorwärts-Schwenkung um einen solchen Winkel 360°-ϕ auszuführen, dass die Vorwärts-Schwenkung von Schritt b) und die weitere Vorwärts-Schwenkung sich zu einer vollen Drehung bzw. Schwenkung der Verschiebeeinrichtung von insgesamt 360° ergänzen.

Bevorzugt ist die Zuführeinrichtung nach Entfernen des Objektes aus dem Startbereich in einem Schritt g) imstande, ein weiteres Objekt auf den Startbereich der Unterlage zu werfen, fallen zu lassen, aufzulegen oder auf sonstige Weise lose auf der Unterlage (U) anzuordnen, wobei die Vorrichtung anschließend in einem Schritt h) imstande ist, die Schritte b) bis f) für das weitere Objekt völlig entsprechend zu wiederholen und auf diese Weise den zu bestimmenden physikalischen oder chemischen Parameter des weiteren Objekts ebenfalls mittels der Prüfeinrichtung zu erfassen. Bevorzugt ist die erfindungsgemäße Vorrichtung imstande, auch diese Schritte selbsttätig bzw. automatisch auszuführen.

Gemäß einer bevorzugten Ausführungsform der Erfindung sind diese Schritte g) und h) für weitere Objekte mehrmals nacheinander wiederholbar.

Gemäß einer Ausführungsform sind die Objekte oder deren Überreste jeweils nach Ausführung des Schrittes c) aus dem Erfassungsbereich der Prüfeinrichtung entfernbar, vorzugsweise automatisch.

Gemäß einer Ausführungsform ist die Verschiebeeinrichtung imstande, das Objekt oder dessen Überreste nach Ausführung des Schrittes c) jeweils durch Wiederholung des Schrittes b) dem Erfassungsbereich wenigstens einer weiteren Prüfeinrichtung oder einem Zwischenlager, Zwischenbehälter oder Sammelbehälter zuzuführen. Bevorzugt weist die Vorrichtung wenigstens eine weitere Prüfeinrichtung auf, in deren Erfassungsbereich das Objekt durch die Verschiebeeinrichtung verschiebbbar ist, wobei jede der weiteren Prüfeinrichtungen jede imstande ist, wenigstens einen physikalischen oder chemischen Parameter der Objekte zu erfassen. Die Verschiebeeinrichtung ist also bevorzugt imstande, das Objekt in den Erfassungsbereich der wenigstens einen weiteren Prüfeinrichtung hinein zu verschieben.

Gemäß einer Ausführungsform ist die Prüfeinrichtung oder die weitere Prüfeinrichtung oder eine der weiteren Prüfeinrichtungen imstande, das Objekt zu zerstören. Die hierbei ausgeführte Prüfung kann z.B. in der Messung der Kraft bestehen, welche erforderlich ist, um das Objekt zu zerstören.

Bevorzugt weist die Verschiebeeinrichtung wenigstens zwei vertikale, voneinander beabstandete Durchbrüche, insbesondere Durchgangsbohrungen, auf, welche je ein Objekt bzw. eines der Objekte aufzunehmen imstande sind.

Bevorzugt ist die Zuführeinrichtung imstande, im Schritt a) ein Objekt bzw. eines der Objekte in einen der Durchbrüche einzuführen, so dass sich dieses Objekt während der Ausführung des Schrittes b) innerhalb dieses Durchbruches befindet.

Ein anderer der Durchbrüche kann so angeordnet sein, dass das Objekt bei Ausführung des Schrittes f) in dem anderen Durchbruch aufgenommen wird, so dass das Objekt sich bei erneuter Ausführung des Schrittes b) innerhalb dieses Durchbruches befindet.

Bevorzugt ist derjenige der Durchbrüche, in welchen das Objekt bei Ausführung des Schrittes f) aufgenommen wird, oben geschlossen oder oben abgedeckt, insbesondere durch eine Glasplatte, so dass ein in diesem Durchbruch aufgenommenes Objekt vor Luftzug und Luftkonvektion geschützt ist. Dies ist vorteilhaft, weil Luftzug oder z.B. thermisch bedingte Luftkonvektion die Prüfung des Objekts, insbesondere seine Wägung, stören oder verfälschen kann.

Die Verschiebeeinrichtung weist bevorzugt eine Schiebekante oder Schiebfläche auf, welche bei Vorwärts-Schwenkung der Verschiebeeinrichtung eine Schubkraft auf das Objekt ausübt und dieses hierdurch verschiebt.

Die Schiebekante oder Schiebfläche verläuft bevorzugt nicht radial zur Schwenkachse, so dass bei der Verschiebung des Objektes durch die Schiebekante oder Schiebefläche reibungsbedingt eine Kraftkomponente nach innen in Richtung der Schwenkachse oder nach außen in einer von der Schwenkachse weggerichteten Richtung auf das Objekt einwirkt.

Gemäß einer bevorzugten Ausführungsform der Erfindung weist die Verschiebeeinrichtung eine Schiebekante oder Schiebfläche auf, welche bei Vorwärts-Schwenkung der Verschiebeeinrichtung eine Schubkraft auf das Objekt ausübt und dieses hierdurch verschiebt, wobei die Schiebekante oder Schiebfläche nicht radial zur Schwenkachse verläuft, so dass bei der Verschiebung des Objektes durch die Schiebekante oder Schiebefläche reibungsbedingt eine Kraftkomponente nach innen in Richtung der Schwenkachse oder nach außen in einer von der Schwenkachse weggerichteten Richtung auf das Objekt einwirkt.

Bevorzugt weist eine der Prüfeinrichtungen einen Stempel auf, welcher zum Zweck der Bestimmung der horizontalen Ausdehnung und/oder der Härte des Objektes parallel zur Unterlage und parallel zur Schiebekante oder Schiebefläche verfahrbar ist.

Gemäß einer Ausführungsform ist die Unterlage teilweise durch einen Tisch gebildet, auf welchen das Objekt mittels der Verschiebeeinrichtung aufschiebbar ist, wobei der Tisch gegenüber einem Anschlag oder gegenüber einem Abstreifer in solcher Weise linear verfahrbar ist, dass das auf den Tisch aufgeschobene Objekt reibungsbedingt an einer bestimmten Position positioniert und in dem fall, dass das Objekt eine längliche Form aufweist, in einer bestimmten Richtung ausgerichtet wird.

Die Unterlage kann teilweise durch einen Tisch gebildet sein, auf welchen das Objekt oder seine Überreste mittels der Verschiebeeinrichtung aufschiebbar sind und unter welchem sich ein Hohlraum befindet, wobei die Vorrichtung einen oberhalb des Tisches angeordneten Abstreifer aufweist und wobei der Tisch gegenüber dem Abstreifer in solcher Weise linear verfahrbar ist, dass das Objekt oder dessen Überreste vom Tisch abgestreift werden und in den Hohlraum fallen. Der Abstreifer kann insbesondere durch die Schiebefläche gebildet sein.

Gemäß einer Ausführungsform weist die erfindungsgemäße Vorrichtung einen Aufprallsensor auf, welcher das Auftreffen eines Objekts auf den Startbereich registriert und daraufhin die Ausführung des Schrittes b) auslöst, bzw. welcher das Auftreffen eines der Objekte auf den Startbereich zu registrieren und daraufhin die Ausführung des Schrittes b) auszulösen imstande ist.

Gemäß einer Ausführungsform ist die Verschiebeeinrichtung nach Ausführung des Schrittes b) und vor Ausführung des Schrittes d) oder vor Ausführung des Schrittes e) zunächst um einen bestimmten Zusatz-Vorwärts-Schwenkwinkel dϕ vorwärts schwenkbar und danach um einen dem Zusatz-Vorwärts-Schwenkwinkel dϕ entgegengesetzt gleichen Zusatz-Rückwärts-Schwenkwinkel -dϕ rückwärts schwenkbar.

Der Zusstz-Vorwäts-Schwenkwinkel dϕ kann insbesondere gleich dem halben Öffnungswinkel sein, unter dem der Erfassungsbereich von der Schwenkachse aus gesehen erscheint.

Die Zuführeinrichtung kann ein Vorratsgefäß mit wenigstens einer Öffnung aufweisen, in welchem die Objekte vor Ausführung des Schritts a) angeordnet sind, wobei die Zuführeinrichtung über dem Zwischengehälter angeordnet ist und die Zuführeinrichtung oder das Vorratsgefäß so schwenkbar sind, dass die Öffnung nach unten weist, wodurch die Objekte direkt oder über eine Rutsche in den Zwischenbehälter fallen.

Die Verschiebeeinrichtung kann gegen eine solche mit anderen Abmessungen auswechselbar sein.

Der Tisch und der Abstreifer können Bestandteile einer erfindungsgemäßen Anordnung zum Abstreifen wenigstens eines Objektes oder von Material von einer Oberfläche sein.

Gemäß einer alternativen Ausführungsform der Erfindung führt die Verschiebeeinrichtung anstelle der Schritte e) und f) mittels des Antriebes eine der Verschiebebewegung nicht entgegengesetzt gleiche Rückkehrbewegung aus, während welcher die Verschiebeeinrichtung das Objekt weder verschiebt noch berührt, und nach welcher sich die Verschiebeeinrichtung wieder in der Ausgangsstellung befindet. Die Verschiebeeinrichtung führt also gemäß dieser alternativen Ausführungsform die der Verschiebebewegung entgegengesetzt gleiche Gegenbewegung von Schritt e) nicht durch, sondern eine andere Bewegung.

Bevorzugt ist die erfindungsgemäße Vorrichtung imstande, die genannten Schritte oder wenigstens einen Teil derselben selbsttätig bzw. automatisch auszuführen.

Die Aufgabe wird ferner erfindungsgemäß gelöst durch ein Verfahren zur Bestimmung wenigstens eines physikalischen oder chemischen Parameters, wie Gewicht, Härte, Dicke, Länge, Dufchmesser, Löslichkeit, Schadstoffkonzentration, von Objekten von fester oder gelartiger Konsistenz, insbesondere von pharmazeutischen Objekten, wie Tabletten, Pillen oder Oblongs, unter Verwendung einer Zuführeinrichtung, einer Unterlage, einer Verschiebeeinrichtung, mindestens einer Prüfeinrichtung, wie Waage, Härteprüfer oder Längenmeßeinrichtung, sowie eines Hub- und Senkmechanismus, welcher imstande ist, den vertikalen Abstand zwischen Verschiebeeinrichtung und Unterlage zu verändern, wobei die Verschiebeeinrichtung zunächst oberhalb der Unterlage in einer Anfangsstellung angeordnet wird, in welcher der lichte vertikale Abstand zwischen Verschiebeeinrichtung und Unterlage kleiner ist als die Vertikalausdehnung des Objektes oder den Wert Null aufweist, und danach folgende Schritte ausgeführt werden:
a) mittels der Zuführeinrichtung wird eines der Objekte auf einen vorgegebenen Startbereich der Unterlage geworfen, fallengelassen, aufgelegt oder in sonstige Weise lose auf der Unterlage angeordnet,
b) die Verschiebeeinrichtung wird mittels eines Antriebes einer Verschiebebewegung unterworfen hierdurch dieses Objekt unter Entfernung desselben aus dem Startbereich auf der Unterlage in den Erfassungsbereich der Prüfeinrichtung verschoben, so dass diese imstande ist, im Schritt c) den oder wenigstens einen der zu bestimmenden physikalischen oder chemischen Parameter dieses Objekts zu erfassen,
c) der oder wenigstens einer der zu bestimmenden physikalischen oder chemischen Parameter dieses Objekts werden mittels der Prüfeinrichtung erfaßt,
d) mittels des Hub- und Senkmechanismus wird ein lichter vertikaler Mindestabstand zwischen Unterlage und Verschiebeeinrichtung hergestelllt, welcher größer ist als die Vertikalausdehnung d des Objektes, wobei das Objekt auf der Unterlage verbleibt,
e) die Verschiebeeinrichtung wird anschließend mittels des Antriebes einer der Verschiebebewegung entgegengesetzt gleichen Gegenbewegung unterworfen, wobei den Mindestabstand nicht unterschritten und somit das Objekt verschoben oder berührt wird, und
f) mittels des Hub- und Senkmechanismus wird anschließend der lichte vertikale Abstand zwischen Verschiebeeinrichtung und Unterlage auf den Wert Null oder auf einen Wert, welcher kleiner ist als die Vertikalausdehnung des Objektes, vermindert und somit die Verschiebeeinrichtung gegenüber der Unterlage in die Anfangsstellung zurückgebracht,
wobei als Antrieb ein Schwenkantrieb verwendet wird und die Verschiebebewegung sowie die Gegenbewegung jeweils Schwenkbewegungen sind.

Das erfindungsgemäße Verfahren beinhaltet daher die Bestimmung wenigstens eines physikalischen oder chemischen Parameters, wie Gewicht, Härte, Dicke, Länge, Durchmesser, Löslichkeit, Schadstoffkonzentration, von Objekten von fester oder gelartiger Konsistenz, insbesondere von pharmazeutischen Objekten, wie Tabletten, Pillen oder Oblongs, unter Verwendung einer Zuführeinrichtung, einer Unterlage, einer Verschiebeeinrichtung, mindestens einer Prüfeinrichtung, wie Waage, Härteprüfer oder Längenmeßeinrichtung, sowie eines Hub- und Senkmechanismus, welcher imstande ist, den vertikalen Abstand zwischen Verschiebeeinrichtung und Unterlage zu verändern, wobei die Verschiebeeinrichtung zunächst oberhalb der Unterlage in einer Anfangsstellung angeordnet wird, in welcher der lichte vertikale Abstand zwischen Verschiebeeinrichtung und Unterlage kleiner ist als die Vertikalausdehnung des Objektes oder den Wert Null aufweist, und danach folgende Schritte ausgeführt werden:
a) mittels der Zuführeinrichtung wird eines der Objekte auf einen vorgegebenen Startbereich der Unterlage geworfen, fallengelassen, aufgelegt oder in sonstige Weise lose auf der Unterlage angeordnet,
b) die Verschiebeeinrichtung wird mittels eines Antriebes einer Verschiebebewegung unterworfen hierdurch dieses Objekt unter Entfernung desselben aus dem Startbereich auf der Unterlage in den Erfassungsbereich der Prüfeinrichtung verschoben, so dass diese imstande ist, im Schritt c) den oder wenigstens einen der zu bestimmenden physikalischen oder chemischen Parameter dieses Objekts zu erfassen,
c) der oder wenigstens einer der zu bestimmenden physikalischen oder chemischen Parameter dieses Objekts werden mittels der Prüfeinrichtung erfaßt,
d) mittels des Hub- und Senkmechanismus wird ein lichter vertikaler Mindestabstand zwischen Unterlage und Verschiebeeinrichtung hergestelllt, welcher größer ist als die Vertikalausdehnung des Objektes, wobei das Objekt auf der Unterlage verbleibt,
e) die Verschiebeeinrichtung wird anschließend mittels des Antriebes einer der Verschiebebewegung entgegengesetzt gleichen Gegenbewegung unterworfen, wobei den Mindestabstand nicht unterschritten und somit das Objekt verschoben oder berührt wird, und
f) mittels des Hub- und Senkmechanismus wird anschließend der lichte vertikale Abstand zwischen Verschiebeeinrichtung und Unterlage auf den Wert Null oder auf einen Wert, welcher kleiner ist als die Vertikalausdehnung des Objektes, vermindert und somit die Verschiebeeinrichtung gegenüber der Unterlage in die Anfangsstellung zurückgebracht.

Als Antrieb wird ein Schwenkantrieb verwendet, wobei die Verschiebebewegung sowie die Gegenbewegung jeweils Schwenkbewegungen sind.

Gemäß einer bevorzugten Verfahrensvariante wird die Verschiebeeinrichtung
- im Schritt b) mittels des Schwenkantriebes als Verschiebebewegung in einer vorgegebenen Drehrichtung einer Vorwärts-Schwenkung von einer Anfangs- in eine Endstellung um einen vorgegebenen Vorwärts-Schwenkwinkel ϕ um eine vertikale oder zur Unterlage senkrechte Schwenkachse unterworfen und hierdurch dieses Objekt unter Entfernung desselben aus dem Startbereich auf der Unterlage in den Erfassungsbereich der Prüfeinrichtung verschoben, so dass diese imstande ist, in einem Schritt c) den oder wenigstens einen der zu bestimmenden physikalischen oder chemischen Parameter dieses Objekts zu erfassen,
- und im Schritt e) mittels des Schwenkantriebes als Gegenbewegung entgegen der vorgegebenen Drehrichtung einer Rückwärts-Schwenkung um einen dem Vorwärts-Schwenkwinkel entgegengesetzt gleichen Rückwärts-Schwenkwinkel unterzogen, wobei der Mindestabstand nicht unterschritten und somit das Objekt nicht verschoben oder berührt wird.

Alternativ hierzu kann die Verschiebeeinrichtung im Schritt e) mittels des Schwenkantriebes anstelle der Rückwärts-Schwenkung statt dessen einer weiteren Vorwärts-Schwenkung, nämlich einer Zusatz-Vorwärts-Schwenkung, und zwar um einen Winkel 360°-ϕ, unterzogen werden, wobei der Mindestabstand nicht unterschritten und somit das Objekt nicht verschoben oder berührt wird. Diese Zusatz-Vorwärts-Schwenkung ergänzt sich mit der Vorwärts-Schwenkung von Schritt b) somit zu genau einer vollen Drehung bzw. Schwenkung der Verschiebeeinrichtung um insgesamt 360°.

Gemäß einer alternativen Variante wird daher die Verschiebeeinrichtung im Schritt e) mittels des Schwenkantriebes anstelle der Gegenbewegung bzw. der Rückwärts-Schwenkung einer weiteren Vorwärts-Schwenkung um einen solchen Winkel 360°-ϕ unterzogen, dass die Vorwärts-Schwenkung von Schritt b) und die weitere Vorwärts-Schwenkung sich zu einer vollen Drehung bzw. Schwenkung der Verschiebeeinrichtung von insgesamt 360° ergänzen.

Gemäß einer Variante wird
g) mittels der Zuführeinrichtung nach Entfernen des Objektes aus dem Startbereich ein weiteres Objekt auf den Startbereich der Unterlage geworfen, fallengelassen, aufgelegt oder auf sonstige Weise lose auf der Unterlage angeordnet, und
h) anschließend die Schritte b) bis f) für das weitere Objekt völlig entsprechend wiederholt und auf diese Weise der zu bestimmende physikalische oder chemische Parameter des weiteren Objekts ebenfalls mittels der Prüfeinrichtung erfaßt.

Insbesondere können die Schritte g) und h) für weitere Objekte mehrmals nacheinander wiederholt werden.

Gemäß einer Variante werden die Objekte oder deren Überreste jeweils nach Ausführung des Schrittes c) aus dem Erfassungsbereich der Prüfeinrichtung entfernt werden, insbesondere z.B. um bei der Prüfung des nächsten Objekts Verfälschungen des Prüfergebnisses zu vermeiden.

Gemäß einer Variante werden das Objekt oder dessen Überreste nach Ausführung des Schrittes c) jeweils durch Wiederholung des Schrittes b) mittels der Verschiebeeinrichtung dem Erfassungsbereich wenigstens einer weiteren Prüfeinrichtung oder einem Zwischenlager, Zwischenbehälter oder Sammelbehälter zugeführt. Das Objekt kann somit mittels der Verschiebeeinrichtung in den Erfassungsbereich einer weiteren oder nacheinander in den Erfassungsbereich mehrerer weiterer Prüfeinrichtungen verschoben werden, wobei mittels jeder derselben wenigstens ein physikalischer oder chemischer Parameter des Objekts erfaßt wird.

Gemäß einer Variante wird das Objekt durch die Prüfeinrichtung oder eine der weiteren Prüfeinrichtungen zerstört, beispielsweise zum Zweck der Bestimmung der Bruchkraft.

Bevorzugt weist die Verschiebeeinrichtung wenigstens zwei vertikale voneinander beabstandete Durchbrüche, insbesondere Durchgangsbohrungen, auf, in welchen je ein Objekt aufgenommen wird.

Im Schritt a) kann ein Objekt bzw. eines der Objekte mittels der Zuführeinrichtung in einen der Durchbrüche eingeführt werden, so dass sich das Objekt während der Ausführung des Schrittes b) innerhalb dieses Durchbruches befindet.

Das Objekt kann durch Ausführung des Schrittes f) in einem anderen der Durchbrüche aufgenommen werden, so dass sich das Objekt bei erneuter Ausführung des Schrittes b) innerhalb dieses Durchbruches befindet.

Bevorzugt ist hierbei derjenige der Durchbrüche, in welchen das Objekt bei Ausführung des Schrittes f) aufgenommen wird, oben geschlossen oder oben abgedeckt, insbesondere durch eine Glasplatte.

Gemäß einer Verfahrensvariante weist die Verschiebeeinrichtung eine Schiebekante oder Schiebfläche auf, mittels welcher das Objekt bei Vorwärts-Schwenkung der Verschiebeeinrichtung verschoben wird.

Als Verschiebeeinrichtung kann eine solche verwendet werden, deren Schiebekante oder Schiebfläche nicht radial zur Schwenkachse verläuft, so dass bei der Verschiebung des Objektes durch die Schiebekante oder Schiebefläche reibungsbedingt eine Kraftkomponente nach innen in Richtung der Schwenkachse oder nach außen in eine von der Schwenkachse weggerichteten Richtung auf das Objekt ausgeübt wird.

Gemäß einer Verfahrensvariante weist die Verschiebeeinrichtung eine Schiebekante oder Schiebfläche auf, mittels welcher das Objekt bei Vorwärts-Schwenkung der Verschiebeeinrichtung verschoben wird, wobei als Verschiebeeinrichtung eine solche verwendet wird, deren Schiebekante oder Schiebfläche nicht radial zur Schwenkachse verläuft, so dass bei der Verschiebung des Objektes durch die Schiebekante oder Schiebefläche reibungsbedingt eine Kraftkomponente nach innen in Richtung der Schwenkachse oder nach außen in eine von der Schwenkachse weggerichteten Richtung auf das Objekt ausgeübt wird.

Die horizontale Ausdehnung und/oder die Härte des Objektes wird vorzugsweise mittels einer solchen Prüfeinrichtung bestimmt, welche einen Stempel aufweist, welcher parallel zur Unterlage und parallel zur Schiebekante oder Schiebefläche verfahrbar ist.

Gemäß einer vorteilhaften Variante wird als Unterlage eine solche verwendet, welche teilweise durch einen Tisch gebildet ist, auf welchen das Objekt mittels der Verschiebeeinrichtung aufgeschoben wird, wobei der Tisch gegenüber einem Anschlag oder gegenüber einem Abstreifer in solcher Weise linear verfahren wird, dass das auf den Tisch aufgeschobene Objekt reibungsbedingt an einer bestimmten Position positioniert und in dem Fall, dass das Objekt eine längliche Form aufweist, in einer bestimmten Richtung ausgerichtet wird.

Gemäß einer Variante des Verfahrens wird als Unterlage eine solche verwendet, welche teilweise durch einen Tisch gebildet ist, auf welchen das Objekt oder seine Überreste mittels der Verschiebeeinrichtung aufgeschoben werden und unter welchem sich ein Hohlraum befindet, wobei ein oberhalb des Tisches angeordneten Abstreifer verwendet wird, und wobei der Tisch gegenüber dem Abstreifer in solcher Weise horizontal verfahren wird, dass das Objekt oder dessen Überreste vom Tisch abgestreift werden und in den Hohlraum fallen. Insbesondere kann z.B. die Schiebefläche selbst als Abstreifer verwendet werden.

Gemäß einer Variante wird ein Aufprallsensor verwendet, welcher das Auftreffen eines Objekts auf den Startbereich registriert und daraufhin die Ausführung des Schrittes b) auslöst.

Gemäß einer Variante wird die Verschiebeeinrichtung nach Ausführung des Schrittes b) und vor Ausführung des Schrittes d) oder vor Ausführung des Schrittes e) zunächst um einen bestimmten Zusatz-Vorwärts-Schwenkwinkel dϕ vorwärts geschwenkt und danach um einen dem Zusatz-Vorwärts-Schwenkwinkel dϕ entgegengesetzt gleichen Zusatz-Rückwärts-Schwenkwinkel -dϕ rückwärts geschwenkt.

Der Zusatz-Vorwärts-Schwenkwinkel dϕ ist bevorzugt gleich dem halben Öffnungswinkel, unter dem der Erfassungsbereich von der Schwenkachse aus gesehen erscheint.

Zum Abstreifen des Objektes oder seiner Überreste von dem Tisch kann insbesondere ein erfindungsgemäßes Verfahren zum Abstreifen wenigstens eines Objektes oder von Material von einer Oberfläche eingesetzt werden.

Als Verschiebeeinrichtung kann vorteilhaft eine solche verwendet werden, welche gegen eine solche mit anderen Abmessungen auswechselbar ist.

Gemäß einer alternativen Variante des Verfahrens wird anstelle der Schritte e) und f) folgender Schritt ausgeführt: die Verschiebeeinrichtung wird mittels des Antriebes einer der Verschiebebewegung nicht entgegengesetzt gleichen Rückkehrbewegung unterworfen, durch welche die Verschiebeeinrichtung wieder in die Ausgangsstellung gebracht wird, ohne das Objekt zu verschieben oder zu berühren.

Der Abstreifer kann einen Besen oder Borsten aufweisen, welche auf der Oberfläche aufstehen oder aufliegen und mittels welchen das Objekt oder dessen Überreste oder das Material von der Oberfläche abstreifbar sind.

Der Abstreifer kann einen biegsamen Materialstreifen aufweisen, welcher auf der Oberfläche aufsteht oder aufliegt und mittels welchem das Objekt oder dessen Überreste oder das Material von der Oberfläche abstreifbar sind.

Kurzbeschreibung der Zeichnung, in welcher zeigen:
- Fig. 1: einen Draufblick auf eine erfindungsgemäße Vorrichtung, wobei die Zuführeinrichtung, die Verschiebeeinrichtung, der Schwenkantrieb und der Hub- und Senkmechanismus weggelassen sind,
- Fig. 2: eine Draufsicht auf die Verschiebeeinrichtung,
- Fig. 3: einen Draufblick auf eine erfindungsgemäße Vorrichtung mit der in einer Ausgangsstellung auf die Unterlage aufgesetzten Verschiebeeinrichtung,
- Fig. 4: die Vorrichtung von Fig. 3 nach Aufbringen eines Objekts auf den Startbereich, wobei die Zuführeinrichtung mit einer Mehrzahl von Objekten darin sowie ein Rüttelantrieb zusätzlich dargestellt sind, welche in Fig. 3 weggelassen sind,
- Fig. 5: die Situation nach Ende einer Vorwärts-Schwenkbewegung,
- Fig. 6: die Situation nach Ende einer Rückwärts-Schwenkbewegung und nach Aufbringen eines weiteren Objekts auf den Startbereich,
- Fig. 7: die Situation nach Ende einer erneuten Vorwärts-Schwenkbewegung,
- Fig. 8: die Situation nach Ende einer erneuten Rückwärts-Schwenkbewegung und nach Aufbringen eines weiteren Objekts auf den Startbereich, und
- Fig. 9: die Situation nach Ende einer abermaligen Vorwärts-Schwenkbewegung.

Alle Figuren sind schematische Darstellungen, welche sich auf beispielhafte Ausführungsformen der Erfindung beziehen.

Die Figuren beziehen sich auf eine bevorzugte Ausführungsform der erfindungsgemäßen Vorrichtung zur Bestimmung wenigstens eines physikalischen oder chemischen Parameters, wie Gewicht, Härte, Dicke, Länge, Durchmesser, Löslichkeit, Schadstoffkonzentration, von Objekten von fester oder gelartiger Konsistenz, insbesondere von pharmazeutischen Objekten 1,2,3,4,5, wie z.B. Tabletten, Pillen oder Oblongs. Die Vorrichtung umfasst eine Zuführeinrichtung Z, eine Unterlage U, eine über der Unterlage U angeordnete Verschiebeeinrichtung S, mindestens drei Prüfeinrichtungen P,P',P", nämlich eine Waage P, eine Dickenmeßeinrichtung P' sowie eine Längenmeßeinrichtung P", welche mit einem Härteprüfer kombiniert ist, sowie einem Hub- und Senkmechanismus HS, welcher imstande ist, den vertikalen Abstand zwischen Verschiebeeinrichtung S und Unterlage U zu verändern, und einen Schwenkantrieb SA zum Antrieb der Verschiebeeinrichtung S, sowie weitere Komponenten.

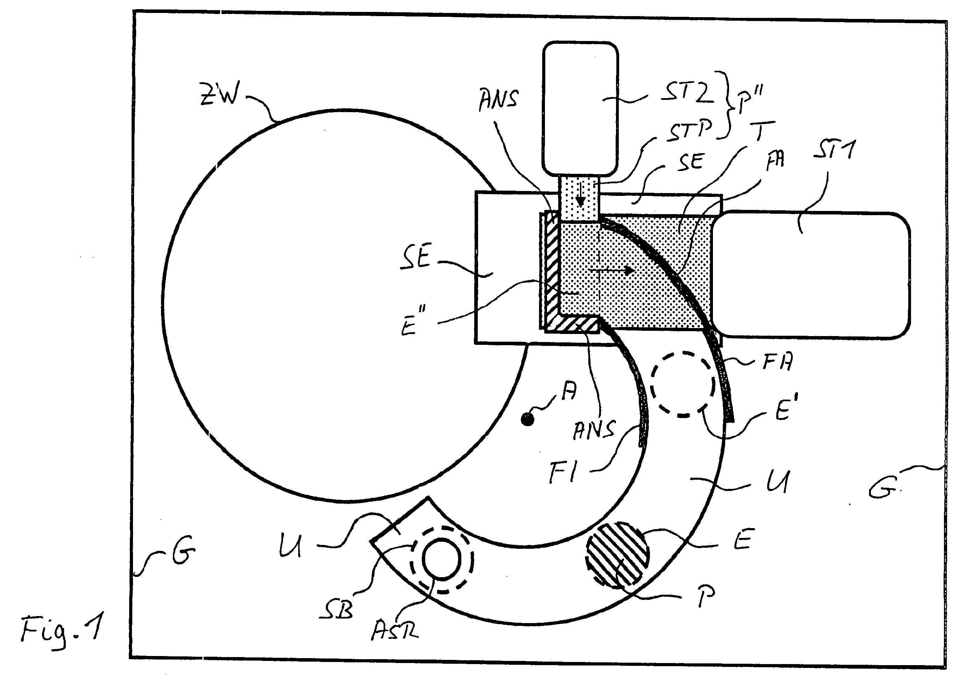

Fig. 1 zeigt schematisch einen Draufblick auf eine erfindungsgemäße Vorrichtung, wobei die Zuführeinrichtung Z, die Verschiebeeinrichtung S, der Schwenkantrieb SA und der Hub- und Senkmechanismus HS weggelassen sind, damit der Blick auf die darunterliegenden Komponenten unbehindert ist. Die Vorrichtung besitzt ein Gehäuse G, in welchem alle übrigen Komponenten angeordnet sind.

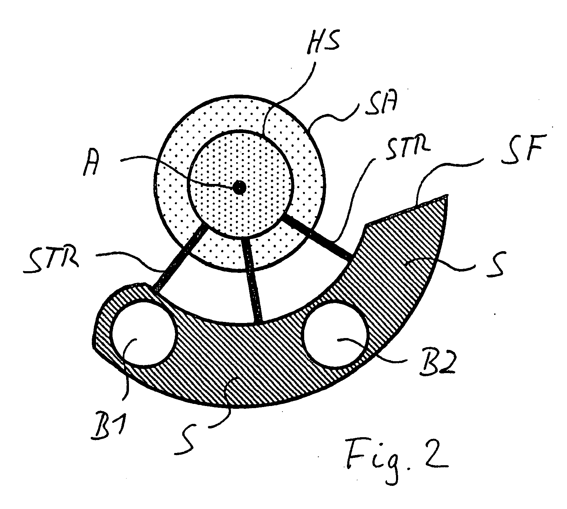

Die Unterlage U besitzt im wesentlichen die Form einer C-förmig um einen Mittelpunkt, nämlich eine Schwenkachse A, gebogenen Platte. Die Schwenkachse A stellt das Drehzentrum von Schwenkbewegungen der Verschiebevorrichtung S (Fig. 2) dar, wie unten näher erläutert wird. Die Unterlage U ist in der hier dargestellten Ausführungsform der Erfindung nicht schwenkbar, sondern starr in dem Gehäuse G montiert.

In einen Endbereich der Unterlage ist ein Aufprallsensor ASR eingelassen, welcher imstande ist zu registrieren, ob ein Objekt auf einen Startbereich, SB auf der Oberseite der Unterlage U auftrifft. Die Oberfläche des Aufprallsensors ASR liegt in einer Ebene mit der Oberfläche der Unterlage U. Die Funktion des Aufprallsensors ASR wird unten noch näher erläutert.

Von dem Startbereich SB beabstandet befindet sich auf der Unterlage U ferner ein Erfassungsbereich E, welcher gegeben ist durch den Bereich, in welchem ein zu prüfendes Objekt liegen muß, damit sein Gewicht durch eine Prüfeinrichtung P, nämlich eine Präzisionswaage P, erfaßt werden kann. Die Oberfläche der Waage P liegt in einer Ebene mit der Oberfläche der Unterlage U.

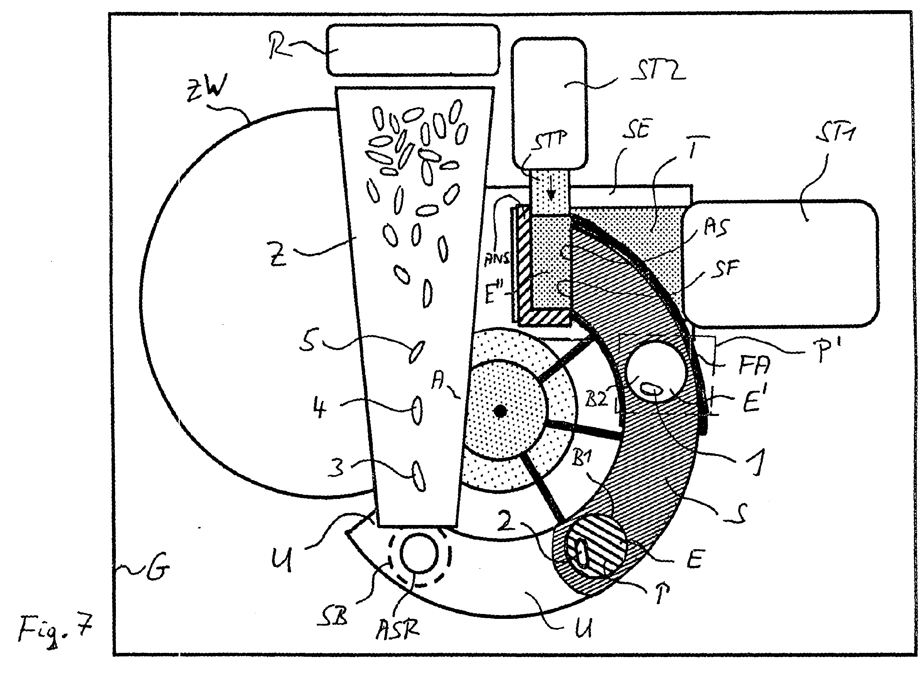

Vom Erfassungsbereich E beabstandet befindet sich auf der Unterlage U ferner ein Erfassungsbereich E', welcher gegeben ist durch den Bereich, in welchem ein zu prüfendes Objekt liegen muß, damit seine Dicke, d.h. seine vertikale Ausdehnung, durch eine weitere Prüfeinrichtung P', nämlich einen Dickenmesser P', erfaßt werden kann. Der nur in Figur 7 (dort gestrichelt und durchsichtig) dargestellte Dickenmesser P' befindet sich über der Unterlage U und ist imstande, einen Stempel so weit nach unten auszufahren, bis dieser ein im Erfassungsbereich E' auf der Unterlage U liegendes Objekt berührt, und aus dem Verfahrweg des Stempels die Dicke des Objekts zu bestimmen.

Vom Erfassungsbereich E' beabstandet befindet sich auf der Unterlage U ferner ein Erfassungsbereich E", welcher gegeben ist durch den Bereich, in welchem ein zu prüfendes Objekt liegen muß, damit seine horizontale Ausdehnung und seine Härte durch die weitere Prüfeinrichtung P" erfaßt werden können.

Der Abstand des Erfassungsbereichs E vom Startbereich SB ist im Beispiel der Vorrichtung von Figur 1-9 ebenso groß wie der Abstand des Erfassungsbereichs E' vom Erfassungsbereich E. Ebenso sind in diesem Beispiel der Startbereich SB sowie die Erfassungsbereiche E', E" alle gleich weit von der Schwenkachse A entfernt.

Die Unterlage U ist teilweise, nämlich in ihrem dem Startbereich SB gegenüberliegenden Endbereich, durch einen Tisch T gebildet, dessen Oberfläche in einer Ebene mit der übrigen Oberfläche der Unterlage U liegt. Der Tisch T ist mittels eines Stelltriebs ST1 in Richtung des in Fig. 1 nach rechts weisenden Pfeiles einfahrbar und danach wieder in umgekehrter Richtung ausfahrbar. Unter dem Tisch befindet sich ein Hohlraum, dessen Boden durch eine schiefe Ebene SE gebildet ist. Diese fällt (in Fig. 1 nach links) steil in Richtung eines Zwischenbehälters ZW ab. Der Erfassungsbereich E" befindet sich vollständig auf dem Tisch T.

Die weitere Prüfeinrichtung P" befindet sich im Bereich des Tisches T und dient dazu, zunächst die horizontale Ausdehnung und danach die Härte von Objekten zu ermitteln. Die Prüfeinrichtung P" umfasst einen Stelltrieb ST2 sowie einen Stempel STP, welcher mittels des Stelltriebs ST2 in Richtung des in Fig. 1 abwärts weisenden Pfeiles ausfahrbar ist und danach in umgekehrter Richtung wieder einfahrbar ist.

Die Oberfläche des Tisches T ist vorzugsweise eben ausgebildet, insbesondere deswegen, weil hier durch Vorschriften erfüllt werden, welche vorschreiben, dass das Objekt während des Tests seiner Härte oder Bruchkraft auf einer ebenen Fläche zu liegen hat.

Im Bereich des Tisches T befindet sich ferner ein winkelförmiger Anschlag ANS, welcher bei ausgefahrenem Tisch die Bewegungsfreiheit eines auf dem Tisch T liegenden Objekts in zwei Richtungen begrenzt, nämlich in die Ausfahrrichtung des Tisches T und in die Ausfahrrichtung des Stempels STP. Der Anschlag ANS ist starr mit der Unterlage U verbunden und liegt mit seiner Unterkante auf der Tischoberfläche auf.

Durch hinreichend weites Ausfahren des Stempels STP wird ein zwischen Stempel STP und Anschlag ANS befindliches Objekt zwischen dem Stempel STP und dem Anschlag ANS eingeklemmt. Hierdurch erhöht sich die Rückstellkraft auf den Stempel STP, was zur Messung der Horizontalausdehnung des Objekts ausgenutzt wird. Anschließend wird der Stempel STP noch weiter ausgefahren, bis das Objekt, insbesondere wenn es sich um ein hartes Objekt handelt, zerstört wird, wodurch die Rückstellkraft schlagartig nachläßt. Dies wird von der weiteren Prüfeinrichtung P" zur Messung der Härte des Objekts ausgenutzt. Bei weichen oder elastischen Objekten nimmt die Rückstellkraft mit zunehmendem Ausfahrweg weiter zu, was ebenfalls zur Messung der Härte bzw. der Elastizität des Objekts ausgenutzt werden kann; hierbei braucht es nicht zur Zerstörung des Objekts zu kommen.

Oberhalb des Tisches T ist ein Abstreifer AS angeordnet, welcher nicht an der Verfahrbewegung des Tisches T teilnimmt und dessen Unterkante ebenfalls auf der Oberfläche des Tisches T aufliegt. Wird nun der Tisch T in Richtung des nach rechts weisenden Pfeiles verfahren, so werden ein Objekt oder die Überreste eines zerstörten Objekts, welche auf dem Tisch T liegen, durch den Abstreifer AS von der Tischoberfläche abgestreift, fallen auf die schiefe Ebene SE und gleiten auf dieser in den Zwischenbehälter ZW. Somit ist der Tisch T gegenüber dem Abstreifer AS in solcher Weise horizontal verfahrbar, dass ein auf dem Tisch T liegendes Objekt oder, falls dieses zerstört wurde, dessen Überreste vom Tisch T abgestreift werden und in den Hohlraum fallen. Anschließend wird der Tisch T wieder in die in Fig. 1 gezeigte Stellung verfahren.

Gemäß einer abweichenden Ausführungsform liegt die Unterkante des Abstreifers AS nicht auf der Tischoberfläche auf, sondern ist von dieser beabstandet, wobei an der Unterkante des Abstreifers AS ein biegsamer Materialstreifen, Borsten oder ein kleiner Besen angeordnet sind, welche das Objekt oder seine Überreste von der Tischoberfläche abstreifen und auf diese Weise die Tischoberfläche sehr wirksam von Überresten des Objekts säubern.

Gemäß einer weiteren Variante ist ein Teil des Bodens des Zwischenbehälter ZW senkrecht unter dem Tisch T angeordnet, so dass die schiefe Ebene SE entfallen kann.

In ihrem vom Startbereich SB abgewandten Endbereich weist die Unterlage U entlang ihrem Außenrand eine sich von der Oberseite der Unterlage U nach oben erstreckende äußere Führung FA auf, welche in diesem Bereich der Unterlage als Führung für Objekte dient, welche auf der Unterlage in Richtung des Anschlages ANS verschoben werden, wie unten noch näher erläutert wird. Ebenso weist die Unterlage U gegenüber der äußeren Führung FA entlang ihrem Innenrand eine sich von der Oberseite der Unterlage U nach oben erstreckende innere Führung FI auf, welche in diesem Bereich der Unterlage U ebenfalls als Führung für Objekte dient, welche auf der Unterlage in Richtung des Anschlages AS verschoben werden. Somit ist die Unterlage U im Bereich der äußeren Führung FA und der inneren Führung FI rinnenartig ausgebildet.

Die Führungen FA, FI können bis zum Startbereich SB oder darüber hinaus fortgesetzt sein, so dass der von den Führungen FA, FI gesäumte Bereich der Unterlage U rinnenartig ausgebildet ist. Ferner kann die Breite der Verschiebeeinrichtung S so gewählt sein, dass diese auf den Führungen FI, FA aufliegt und nicht auf dem Boden der Rinne, so dass die Führungen FI,FA zugleich als Abstandshalter zwischen der Verschiebeeinrichtung S und dem Boden der Rinne fungieren.

Fig. 2 zeigt schematisch eine Draufsicht auf die Verschiebeeinrichtung S, welche über drei Streben STR starr mit dem Schwenkantrieb SA oder dem Hub- und Senkmechanismus HS verbunden ist. Dieser ermöglicht eine Schwenkbewegung der Verschiebeeinrichtung S um die Schwenkachse A sowohl im als auch entgegen dem Uhrzeigersinn.

Die Verschiebeeinrichtung S besitzt im wesentlichen die Form einer C-förmig gebogenen Platte mit zwei Durchbrüchen B1,B2, welche im vorliegenden Beispiel als Durchgangsbohrungen B1, B2 ausgebildet sind. Der gegenseitige Abstand der Durchgangsbohrungen B1,B2 ist ebenso groß wie der Abstand des Erfassungsbereichs E vom Erfassungsbereich E' und der Abstand vom Startbereich SB zum Erfassungsbereich E. Ebenso sind die Durchgangsbohrungen B1, B2, der Startbereich SB sowie die Erfassungsbereiche E, E', E" alle im wesentlichen gleich weit von der Schwenkachse A entfernt. Der Durchmesser der Durchgangsbohrungen B1,B2 ist nicht größer als derjenige des kleineren der beiden Erfassungsbereiche E, E'.

Oberhalb des Schwenkantriebs SA befindet sich der Hub- und Senkmechanismus HS, welcher auf oder unter dem Schwenkantrieb SA relativ zu diesem drehfest angeordnet ist.

Die Verschiebeeinrichtung S besitzt stirnseitig eine Schiebefläche SF, die im vorliegenden Beispiel nicht radial zur Schwenkachse A verläuft.

Bevorzugt ist die Verschiebeeinrichtung S gegen eine solche mit anderen Abmessungen, z.B. anderer Dicke oder anderer Form oder Größe der Durchbrüche oder anderer Anordnung der Schiebefläche SF auswechselbar. Die Durchbrüche können als Aussparungen ausgebildet sein.

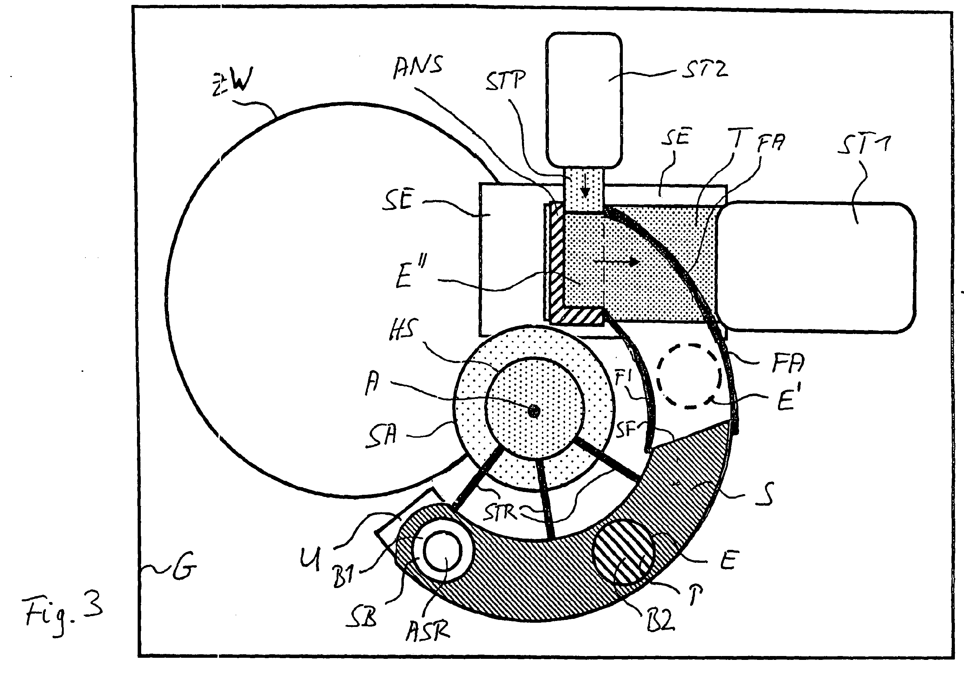

Fig. 3 zeigt schematisch die in einer Ausgangsstellung auf die Unterlage U aufgesetzte Verschiebeeinrichtung S. In der Ausgangsstellung decken sich die Projektionen der Mittelpunkte der Durchgangsbohrung B1 und des Startbereichs SB. Ebenso decken sich die Projektionen der Mittelpunkte der Durchgangsbohrung B2 und des Erfassungsbereichs E. In der Ausgangsstellung befindet sich der Erfassungsbereich E' außerhalb der Projektion der Verschiebeeinrichtung S. Die Zuführeinrichtung ist auch in Fig. 3 weggelassen.

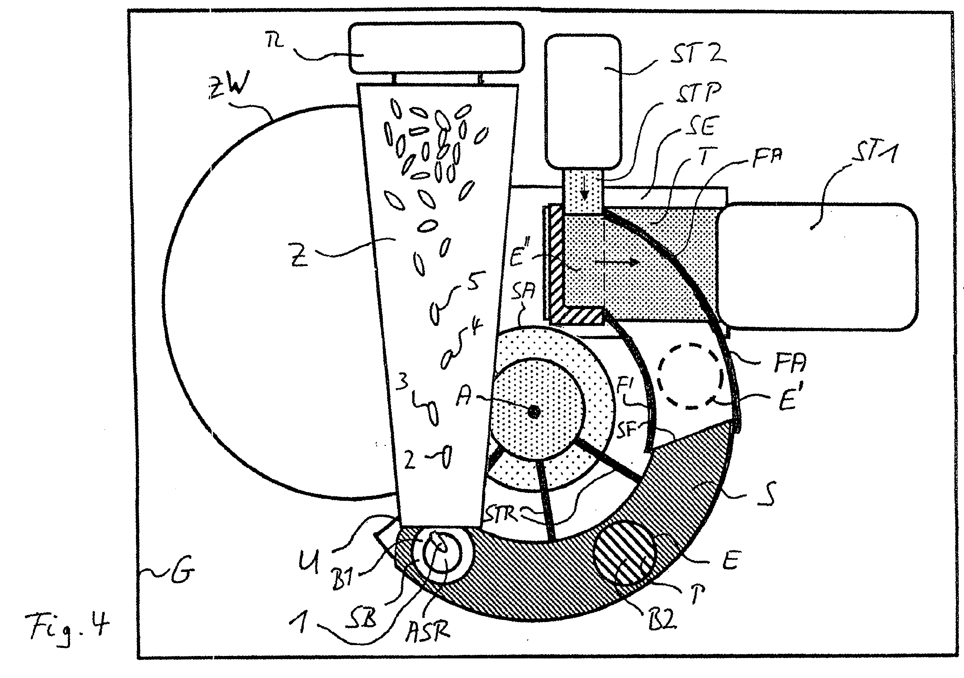

Die Funktion der hier in Rede stehenden Ausführungsform der erfindungsgemäßen Vorrichtung wird nun anhand der weiteren Figuren 4 bis 9 veranschaulicht; hierzu werden im folgenden Schritte a) bis f) erläutert.

Fig. 4 unterscheidet sich von Fig. 3 nur dadurch, dass die Zuführeinrichtung Z mit einer Mehrzahl von Objekten darin sowie ein Rüttelantrieb R zusätzlich dargestellt sind, welche in Fig. 3 aus Gründen der Verständlichkeit weggelassen sind. In der oberhalb des Zwischenbehälters ZW und der Verschiebeeinrichtung S angeordneten schaufelförmigen Zuführeinrichtung Z befinden sich zahlreiche Objekte, nämlich pharmazeutische längliche Tabletten, so genannte Oblongs, darunter auch die mit den Bezugszeichen 2, 3, 4 und 5 bezeichneten Objekte. Die schaufelförmigen Zuführeinrichtung Z bildet somit einen Vorratsraum, in welchem diese Objekte vor Ausführung des Schrittes a) versammelt sind. Die Zuführeinrichtung Z wird mittels des Rüttelantriebs R in bestimmte Bewegungen versetzt, welche in Verbindung mit einem Gefälle der Zuführeinrichtung Z in der vom Rüttelantrieb R abgewandten Richtung dazu führen, dass die Oblongs einzeln nacheinander aus der Zuführeinrichtung in die Durchgangsbohrung B1 hinein und damit auf den Startbereich SB der Unterlage U fallen. In der Situation von Fig. 4 ist bereits ein erstes Objekt 1 auf den Startbereich SB gefallen (Schritt a).

Der Aufprall wurde vom Aufprallsensor ASR registriert mit erstens der Folge, dass der Rüttelantrieb R angehalten wird, damit kein weiteres Objekt aus der Zuführeinrichtung Z fällt, und mit zweitens der weiteren Folge, dass die Verschiebeeinrichtung S in einem Schritt b) mittels des Schwenkantriebes SA in einer vorgegebenen Drehrichtung (in Fig. 4 gegen den Uhrzeigersinn) eine Vorwärts-Schwenkung von der Anfangs- in eine Endstellung um einen vorgegebenen Vorwärts-Schwenkwinkel ϕ die zur Unterlage U senkrechte Schwenkachse A ausführt. Fig. 4 zeigt die Situation vor Beginn dieser Vorwärts-Schwenkbewegung.

Der Winkel ϕ ist so gewählt, dass das Objekt 1 durch die Vorwärts-Schwenkung vom Startbereich SB in den Erfassungsbereich E verschoben wird. Im Schritt b) wird das Objekt 1 daher unter Entfernung desselben aus dem Startbereich SB auf der Unterlage U in den Erfassungsbereich E der Waage P auf der Unterlage U verschoben, wobei es innerhalb der Durchgangsbohrung B1 verbleibt.

Gemäß einer sehr vorteilhaften (nicht dargestellten) Variante wird die Verschiebeeinrichtung S nach Ausführung des Schrittes b) und vor Ausführung des Schrittes e) zunächst um einen bestimmten Zusatz-Vorwärts-Schwenkwinkel dϕ weiter vorwärts geschwenkt und danach um einen dem Zusatz-Vorwärts-Schwenkwinkel dϕ entgegengesetzt gleichen Zusatz-RÜckwärts-Schwenkwinkel - dϕ wieder rückwärts geschwenkt, wobei der Zusatz-Vorwärts-Schwenkwinkel dϕ vorzugsweise gleich dem halben Öffnungswinkel ist, unter dem der Erfassungsbereich E von der Schwenkachse A aus erscheint. Hierdurch wird vorteilhaft erreicht, dass das Objekt 1 näherungsweise in die Mitte des Erfassungsbereichs E verschobnen wird, wo es zunächst liegenbleibt.

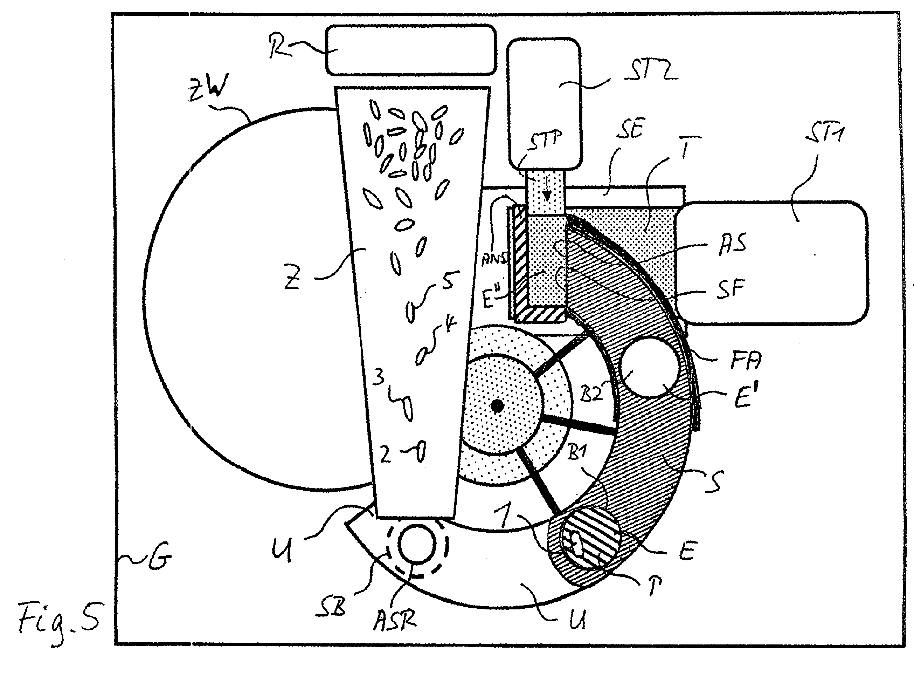

Fig. 5 zeigt die Situation nach Ende der Vorwärts-Schwenkbewegung. Das Objekt 1 befindet sich nun im Erfassungsbereich E der Waage P und wird gewogen, wodurch der physikalische Parameter "Gewicht" bestimmt wird (Schritt c).

Nun stellt der Hub- und Senkmechanismus HS in einem Schritt d) durch Anheben der Verschiebbeinrichtung S einen lichten vertikalen Mindestabstand D zwischen Unterlage U und Verschiebeeinrichtung S her, welcher größer ist als die Vertikalausdehnung d des Objektes 1, wobei das Objekt 1 im Erfassungsbereich E auf der Unterlage U verbleibt.

Anschließend wird die Verschiebeeinrichtung S in einem Schritt e) mittels des Schwenkantriebes SA entgegen der vorgegebenen Drehrichtung um einen dem Vorwärts-Schwenkwinkel ϕ entgegengesetzt gleichen Rückwärts-Schwenkwinkel (-ϕ) zurück geschwenkt, ohne hierbei den Mindestabstand D zu unterschreiten und somit ohne das Objekt 1 zu verschieben oder zu berühren. Somit verbleibt des Objekt weiterhin im Erfassungsbereich E auf der Unterlage U. Gemäß einer hierzu alternativen führt der Schwenkantrieb anstelle des Schrittes e) eine weitere Vorwärts-Schwenkbewegung aus, und zwar um einen Winkel 360°-ϕ , was zur gleichen Stellung der Verschiebeeinrichtung S führt wie der Schritt e).

Danach vermindert der Hub- und Senkmechanismus HS in einem Schritt f) den lichten vertikalen Abstand zwischen Verschiebeeinrichtung S und Unterlage U auf den Wert Null oder auf einen Wert, welcher kleiner ist als die Vertikalausdehnung d des Objektes 1, wodurch die Verschiebeeinrichtung S gegenüber der Unterlage U in die Anfangsstellung zurückkehrt. Hierdurch gerät das Objekt 1 automatisch in die Durchgangsbohrung B2 der Verschiebeeinrichtung S.

Gemäß einer vorteilhaften Variante ist der Durchbruch B2, in welchen das Objekt 1 bei Ausführung des Schrittes f) aufgenommen wurde, oben geschlossen oder oben abgedeckt, insbesondere durch eine Glasplatte (nicht dargestellt), so dass ein in diesem Durchbruch B2 aufgenommenes Objekt 1 vor Luftzug und Luftkonvektion geschützt ist, und die Wägung durch die Waage P erst jetzt, nach dem Schritt f), vorgenommen. Dies ist vorteilhaft, weil Luftzug oder z.B. thermisch bedingte Luftkonvektion die Prüfung des Objekts, insbesondere seine Wägung, stören oder verfälschen kann.

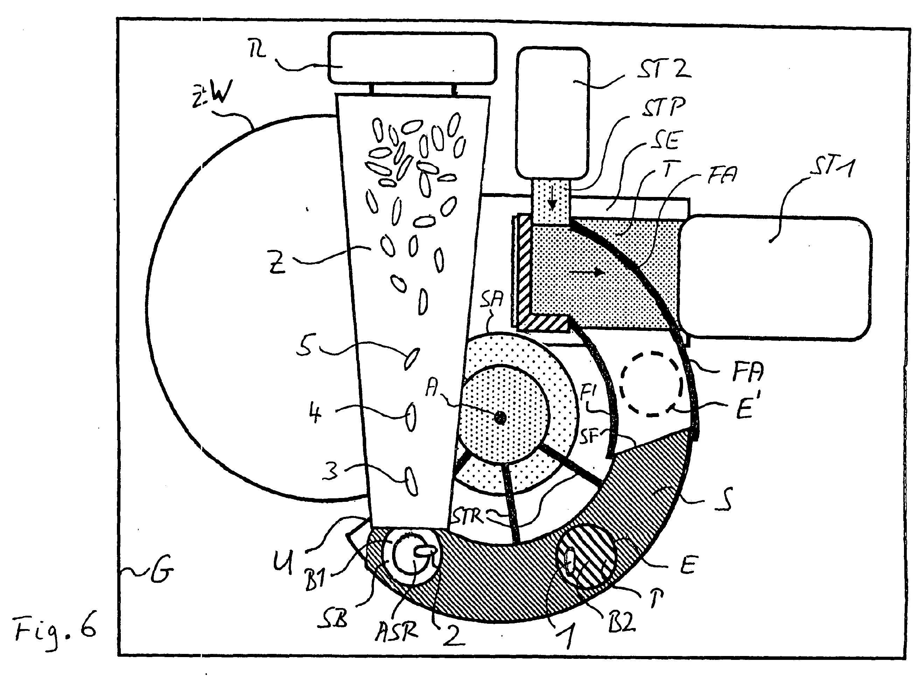

Nun wird unter völlig entsprechender Wiederholung des Schrittes a) ein weiteres Objekt 2 auf den Startbereich SB fallengelassen. Die Situation nach diesem Schritt ist in Fig. 6 gezeigt.

Nun wird völlig entsprechend der Schritt b) wiederholt. Hierdurch werden das Objekt 2 in der Durchgangsbohrung B1 in den Erfassungsbereich E verschoben und gleichzeitig das Objekt 1 in der Durchgangsbohrung B2 in den Erfassungsbereich E' verschoben. Die Situation nach diesem Schritt ist in Fig. 7 gezeigt. Nun wird das Objekt 2 durch die Waage P gewogen (Schritt c); das Objekt 1 wird mittels der Dickenmeßeinrichtung P' einer Dickenmessung unterzogen.

Nun wird der Schritt d) völlig entsprechend wiederholt, d.h. die Verschiebbeinrichtung S wird nach oben von der Unterlage U abgehoben. Danach erfolgt eine erneute Rückwärts-Schwenkung völlig entsprechend dem Schritt e), wobei das Objekt 1 im Erfassungsbereich E' und das Objekt 2 im Erfassungsbereich E liegenbleibt. Danach wird die Verschiebeeinrichtung S unter völlig entsprechender Wiederholung des Schrittes f) angesenkt, wodurch nun das Objekt 2 in die Durchgangsbohrung B2 gerät.

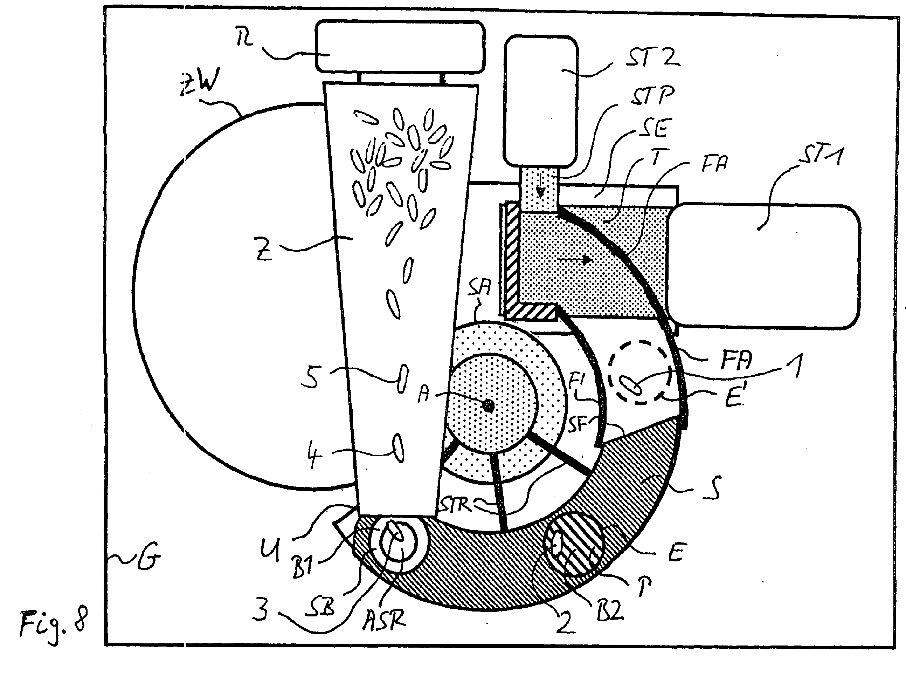

Nun wird unter völlig entsprechender Wiederholung des Schrittes a) ein weiteres Objekt 3 auf den Startbereich SB fallengelassen. Die Situation nach diesem Schritt ist in Fig. 8 gezeigt. Das Objekt 1 befindet sich nun nicht mehr in einer Durchgangsbohrung B1 oder B2 der Verschiebbeinrichtung S.

Nun wird abermals völlig entsprechend der Schritt b) wiederholt. Hierdurch werden das Objekt 3 in den Erfassungsbereich E verschoben (und dort unter erneuter entsprechender Ausführung des Schrittes c gewogen), und gleichzeitig das Objekt 2 in der Durchgangsbohrung B2 in den Erfassungsbereich E' verschoben. Das Objekt 1 wird durch die als Schiebefläche SF ausgebildete Vorderkante SF der Verschiebeeinrichtung S auf der Unterlage U weiter bis in den Erfassungsbereich E" verschoben. Um hierbei ein Herabfallen des Objekts 1 von der Unterlage U zu verhindern und um eine Führung des Objekts 1 zu gewährleisten, weist die Unterlage U im Bereich zwischen den Erfassungsbereichen E' und E" eine innere Führung FI und eine äußere Führung FA auf, welche als nach oben überstehende Seitenwände der Unterlage U so ausgebildet sind, dass sich zwischen ihnen der bei der Vorwärts-Schwenkung führende Teil der Verschiebeeinrichtung S hindurchbewegen kann.

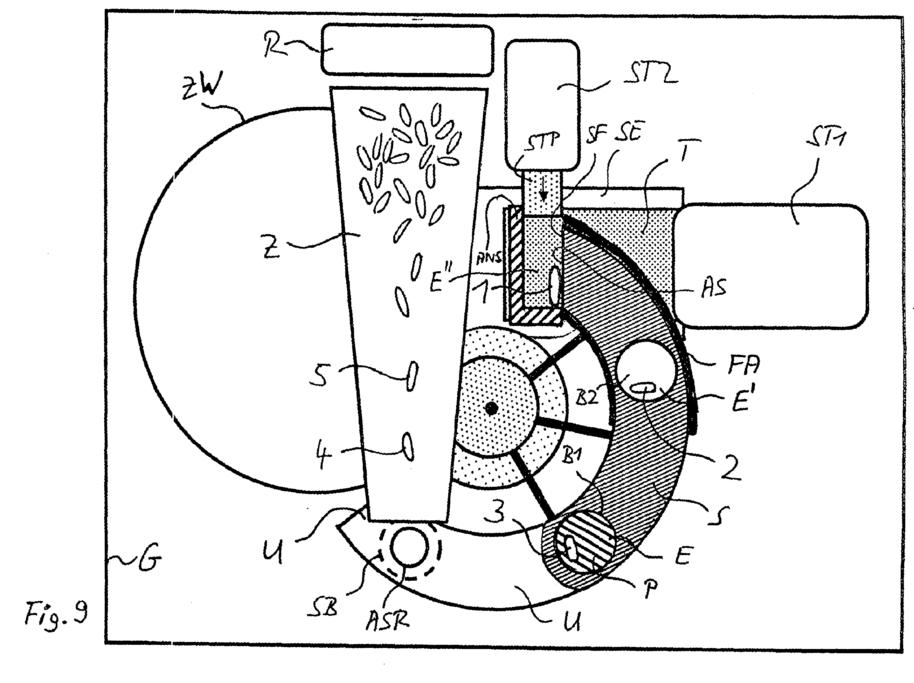

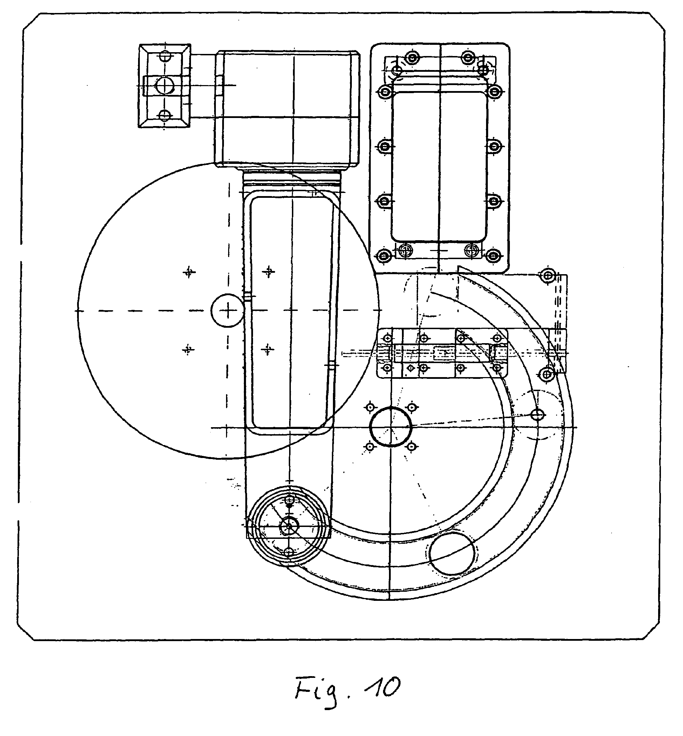

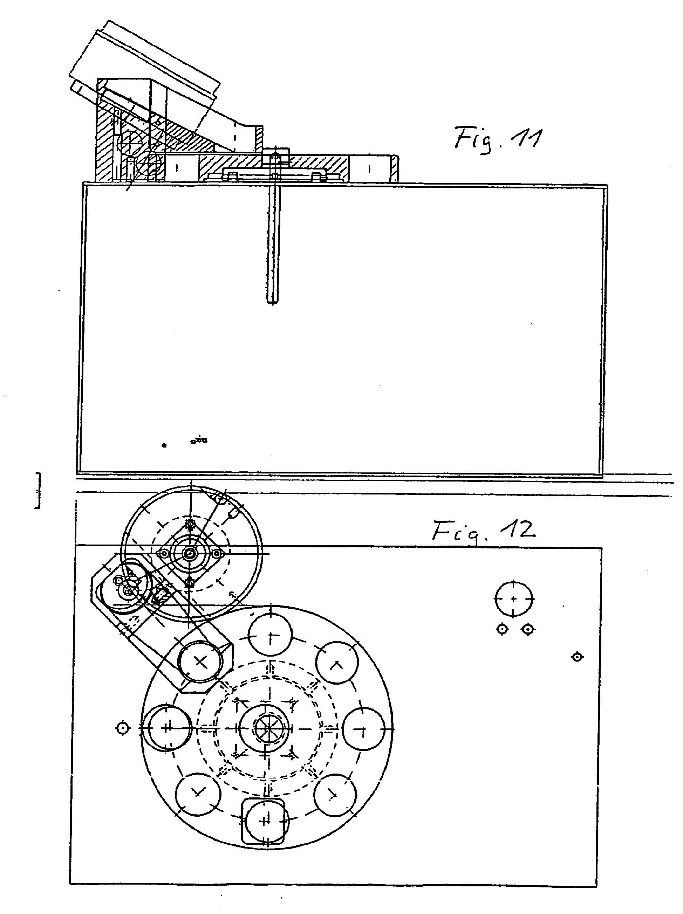

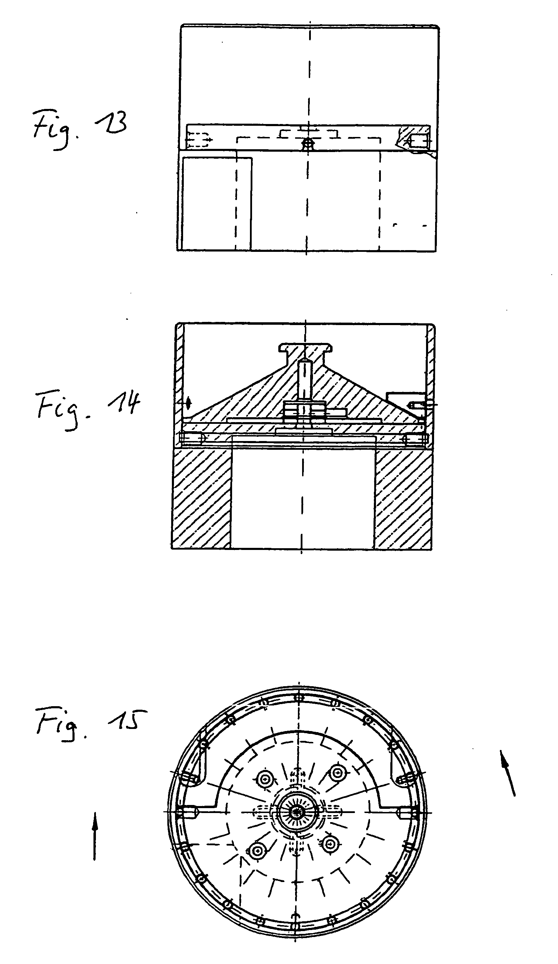

Die Schiebefläche SF verläuft nicht radial zur Schwenkachse A, sondern in einer solchen Richtung, dass bei der Verschiebung des Objektes 1 durch die Schiebefläche SF reibungsbedingt eine Kraftkomponente nach innen in Richtung der Schwenkachse A auf das Objekt 1 ausgeübt wird. Ferner ist die Richtung der Schiebefläche SF so gewählt, dass die Schiebefläche SF unmittelbar nach Ausführung des Schrittes b) parallel zur Verfahrrichtung des Stempels STP verläuft. Auf diese Weise wird vorteilhaft erreicht, dass die Längsachse des länglichen Objekts 1 bei dessen Ankunft im Erfassungsbereich E" von vornherein in Verfahrrichtung des Stempels STP ausgerichtet ist und sich hierbei auch bereits im Bereich des dem Stempel STP gegenüberliegenden Teils des Anschlags ANS befindet. Diese Situation ist in Fig. 9 gezeigt.

Gemäß einer bevorzugten Variante der Erfindung wird vor der Härtemessung der Tisch T in Richtung des nach rechts weisenden Pfeils (Fig. 8) um eine kleine Strecke eingefahren, um das Objekt 1 zusätzlich gegen die Schiebefläche SF zu schieben und damit die Positionierung und Längsausrichtung des auf Härte zu messenden Objekts 1 auf dem Tisch T zu unterstützen bzw. zu verbessern.

Gemäß einer alternativen bevorzugten Variante der Erfindung wird vor der Härtemessung der Tisch T entgegen der Richtung des nach rechts weisenden Pfeils (Fig. 8) um eine kleine Strecke ausgefahren, um das Objekt 1 gegen den Anschlag ANS zu schieben und damit die Positionierung und Längsausrichtung des auf Härte zu messenden Objekts 1 auf dem Tisch T zu unterstützen bzw. zu verbessern.

Nun tritt die Prüfeinrichtung P" in Aktion, indem der Stempel STP mittels des Stelltriebs ST2 ausgefahren wird wie oben beschrieben, wobei zunächst die Länge des Objekts 1 und danach unter Zerstörung des Objekts 1 dessen Härte gemessen wird. Anschließend wird der Tisch T mittels des Stelltriebs ST1 eingezogen wie oben beschrieben, wodurch die Überreste des Objekts 1 auf die schiefe Ebene SE fallen und von dort in den Zwischenbehälter ZW gleiten. Während des Einziehens des Tisches T streift der Abstreifer AS, welcher im vorliegenden Beispiel mit der Schiebefläche SF identisch ist, alle Überreste des Objekts 1 vom Tisch ab, so dass dieser wirkungsvoll gesäubert wird.

Nun werden die Schritte d) bis f) völlig entsprechend neue ausgeführt, usw.. Das Verfahren kann für eine beliebige Zahl von Objekten fortgesetzt werden.

Der Schritt c) braucht nicht notwendigerweise vor dem Schritt d) ausgeführt zu werden; vielmehr kann der Schritt c) statt dessen äquivalent nach jedem der Schritte d), e) und f) ausgeführt werden.

Das Gehäuse G der erfindungsgemäßen Vorrichtung kann wasserdicht oder spritzwasserdicht geschlossen sein, um den Austritt von Abrieb- oder Bruchstaub der Objekte zu verhindern. Dies ist insbesondere dann vorteilhaft, wenn die Objekte giftige oder schädliche Substanzen enthalten. Zudem kann das Gehäuse G Spritzdüsen enthalten, welche Wasser oder ein Reinigungsmittel versprühen können und mittels welchen die gesamte Vorrichtung von Überresten und Abriebstaub der Objekte gereinigt werden kann. Dies ist insbesondere dann vorteilhaft, wenn die Objekte giftige oder schädliche Substanzen enthalten und eine manuelle Reinigung daher nicht ratsam ist.

Ferner kann vorgesehen sein, alle reinigungsrelevanten Teile der Vorrichtung so auszuführen, dass sie ohne Werkzeug entnehmbar sind. Die Waage kann beim Reinigungsvorgang mit einem Stopfen abgedichtet werden oder einen sich automatisch aktivieren Wasserschutz aufweisen.

Bei Verwendung von länglichen Objekten wird gemäß einer weiteren Variante der Tisch T, bevor das im Erfassungsbereich E" liegende Objekt einer Prüfung durch die Prüfeinrichtung P" unterzogen wird, zunächst so weit ausgefahren, dass das Objekt gegen den dem Stellantrieb ST1 gegenüberliegenden Teil des Anschlags ANS geschoben und hierdurch die Längsachse des Objekts in Richtung parallel zur Verfahrrichtung des Stempels STP ausgerichtet wird.

Alternativ hierzu kann bei Verwendung von länglichen Objekten gemäß einer weiteren Variante der Tisch T, bevor das im Erfassungsbereich E" liegende Objekt einer Prüfung durch die Prüfeinrichtung P" unterzogen wird, zunächst so weit eingefahren werden, dass das Objekt gegen die Schiebefläche SF geschoben und hierdurch die Längsachse des Objekts in Richtung parallel zur Verfahrrichtung des Stempels STP ausgerichtet wird.

Gemäß einer weiteren Variante ist der Zwischenbehälter ZW unmittelbar unter dem Tisch T angeordnet, so dass die schiefe Ebene SE entfallen kann und das Objekt oder alle seine Überreste bei Einfahren des Tisches unmittelbar in den Zwischenbehälter ZW fallen.

Der dem Stellantrieb ST1 gegenüberliegenden Teil des Anschlags ANS kann eine der Kontur des Objekts entsprechend geformte Höhlung aufweisen, so dass sich das Objekt zur weiteren Stabilisierung seiner Lage mit einem Teil seiner Außenfläche an den Anschlag ANS anschmiegen kann.

Gemäß einer Ausführungsform ist auf die Verschiebeeinrichtung S im Bereich der Schiebefläche SF eine Kappe anordbar, insbesondere aufsteckbar, deren Stirnseite eine an bestimmte Objekte angepasste Form und/oder Ausrichtung aufweist und an die Stelle der Schiebefläche SF treten kann, so dass eine schnell Anpassung an verschiedene Objektformen oder Objektarten möglich ist.

Gemäß einer Variante weist die Zuführeinrichtung ein Vorratsgefäß mit wenigstens einer Öffnung auf, oder ist die Zuführeinrichtung als Vorratsgefäß mit wenigstens einer Öffnung ausgebildet, in welchem Vorratsgefäß die Objekte 1,2,3,4,5 vor Ausführung des Schritts a) angeordnet sind, wobei die Zuführeinrichtung über dem Zwischengehälter ZW angeordnet ist und so schwenkbar ist, dass die Öffnung nach unten weist, wodurch die Objekte 1,2,3,4,5 direkt oder über eine Rutsche in den Zwischenbehälter ZW fallen.

Im vorliegenden Beispiel ist die Zuführeinrichtung Z schaufelartig ausgebildet und dient als Vorratsgefäß für Objekte, bevor diese einzeln nacheinander auf den Startbereich SB gelangen. Durch Schwenken der Zuführeinrichtung Z um 180° um eine horizontale Achse können die darin befindlichen Objekte in den Zwischenbehälter ZW ausgeleert werden, beispielsweise wenn die Prüfung der Objekte abgebrochen werden soll. Diese Schwenkung kann insbesondere mittels Motorantrieb ferngesteuert erfolgen, so dass hierzu vorteilhafterweise kein manueller Eingriff in die erfindungsgemäße Vorrichtung und in dem in der Praxis häufigen und wichtigen Fall, dass sich die Vorrichtung in einem (z.B. luftdichten) Gehäuse befindet, auch kein Öffnen desselben vonnöten ist.

Ein in der Praxis insbesondere im Langzeitbetrieb sehr bedeutender Vorteil des erfindungsgemäßen Transportes der Objekte durch die Schwenkbewegung der Verschiebeeinrichtung S besteht gegenüber einem linearen Transport darin, dass bei einer Spülung der erfindungsgemäßen Vorrichtung mit Wasser oder Reinigungsmittel eine Abdichtung nur im Bereich der Schwenkachse erforderlich ist. Eine wasser- oder spritzwasserdichte Kapselung des Transportraumes oder Innerraumes bzw. des Gehäuses G der Vorrichtung ist daher bei der erfindungsgemäßen Vorrichtung mit besonders geringem Aufwand und einer wesentlichen Einsparung von Verschleißteilen verbunden, wodurch die Herstellungs- und Unterhaltkosten der Vorrichtung erheblich gesenkt werden und die Gefahr des unbeabsichtigten Austritts von Abrieb oder Staub giftiger, gesundheitsschädlicher oder umweltbelastender Objekte durch Undichtigkeiten, welche z.B. durch den Verschleiß von Dichtungen entstanden sind, vermindert wird.

Die Vorrichtung kann insbesondere bei Anordnung derselben in einem Gehäuse G oder einer Kapselung, selbstreinigend, d.h. mit automatischer Spülung, ausgeführt werden. Sie kann zur Reinigung Spühdüsen enthalten, welche von außen mit Wasser oder Reinigungsflüssigkeit beaufschlagbar sind. Unterhalb des Zwischenbehälters ZW kann ein Abfluß oder Schlauchstutzen für den Austritt des Spülwassers oder der Reinigungsflüssigkeit vorgesehen sein. Die gesamte Vorrichtung kann elektronisch gesteuert sein und zum vollautomatischen Betrieb imstande sein.

Oberhalb des Tisches T ist der Abstreifer AS angeordnet, dessen Unterkante auf der Oberfläche des Tisches T aufliegt. Wird nun der Tisch T in Richtung des nach rechts weisenden Pfeiles verfahren, so werden ein Objekt oder die Überreste eines zerstörten Objekts oder Material, welche auf dem Tisch T liegen, durch den Abstreifer AS von der Tischoberfläche abgestreift und fallen in den darunterliegenden Hohlraum. Somit ist der Tisch T gegenüber dem Abstreifer AS in solcher Weise horizontal verfahrbar, dass ein auf dem Tisch T liegendes Objekt oder, falls dieses zerstört wurde, dessen Überreste, oder auf dem Tisch T liegendes Material vom Tisch T abgestreift werden und in den Hohlraum fallen. Anschließend wird der Tisch wieder in die in Fig. 1 gezeigte Stellung verfahren.

Selbstverständlich braucht der Abstreifer von oben gesehen keine gebogene Form aufzuweisen; eine solche Form kann jedoch bei Verwendung der erfindungsgemäßen Anordnung zum Abstreifen wenigstens eines Objektes oder von Material von einer Oberfläche in einer Vorrichtung zur Bestimmung wenigstens eines physikalischen oder chemischen Parameters von Objekten, insbesondere der Vorrichtung von Fig. 1 bis Fig. 9, sehr vorteilhaft sein.

Gemäß einer abweichenden Ausführungsform liegt die Unterkante des Abstreifers nicht auf der Oberfläche auf, sondern ist von dieser beabstandet, wobei an der Unterkante des Abstreifers ein biegsamer Materialstreifen, Borsten oder ein kleiner Besen angeordnet sind, welche das Objekt oder seine Überreste von der Oberfläche abstreifen und auf diese Weise die Tischoberfläche sehr wirksam von dem Objekt, Überresten des Objekts oder dem Material säubern.

### Gewerbliche Anwendbarkeit:

Die Erfindung ist gewerblich anwendbar z.B. im Bereich der Pharmatechnik.

### Liste der Bezugszeichen:

- 1-5: Objekte, z.B. Oblongs
- A: Schwenkachse
- ANS: Anschlag
- AS: Abstreifer
- ASR: Aufprallsensor
- B: Sammelbehälter
- B1,B2: Durchgangsbohrungen in S
- D: Mindestabstand zwischen T und U bei Ausführung von Schritt e)
- E: Erfassungsbereich von P
- E',E": Erfassungsbereiche von P',P"
- FI, FA: innere Führung, äußere Führung
- G: Gehäuse
- HS: Hub- und Senkmechanismus
- P,P',P": Prüfeinrichtungen
- R: Rüttelantrieb
- S: Verschiebeeinrichtung
- SA: Schwenkantrieb für T
- SB: Startbereich auf U
- SE: schiefe Ebene
- SF: Schiebefläche
- STP: Stempel
- U: Unterlage
- ST1: Stelltrieb für T
- ST2: Stelltrieb für STP
- T: Tisch
- Z: Zuführeinrichtung
- ZW: Zwischenbehälter
- ϕ: Vorwärts-Schwenkwinkel
- -ϕ: Rückwärts-Schwenkwinkel

## Patentansprüche

1. Vorrichtung zur Bestimmung wenigstens eines physikalischen oder chemischen Parameters, wie Gewicht, Härte, Dicke, Länge, Durchmesser, Löslichkeit, Schadstoffkonzentration, von Objekten (1,2,3,4,5) von fester oder gelartiger Konsistenz, insbesondere von pharmazeutischen Objekten, wie Tabletten, Pillen oder Oblongs (1,2,3,4,5), mit einer Zuführeinrichtung (Z), einer Unterlage (U), einer Verschiebeeinrichtung (S), mindestens einer Prüfeinrichtung (P), wie Waage, Härteprüfer oder Längenmeßeinrichtung, sowie einem Hub- und Senkmechanismus (HS), welcher imstande ist, den vertikalen Abstand zwischen Verschiebeeinrichtung (S) und Unterlage zu verändern, wobei
- die Zuführeinrichtung (Z) imstande ist, in einem Schritt a) eines der Objekte (1) auf einen vorgegebenen Startbereich (SB) der Unterlage (U) zu werfen, fallenzulassen, aufzulegen oder in sonstiger Weise lose auf der Unterlage (U) anzuordnen, wobei die Verschiebeeinrichtung (S) oberhalb der Unterlage (U) in einer Anfangsstellung angeordnet ist, in welcher der lichte vertikale Abstand zwischen Verschiebeeinrichtung (S) und Unterlage (U) kleiner ist als die Vertikalausdehnung des Objektes (1) oder den Wert Null aufweist,
- die Verschiebeeinrichtung (S) imstande ist, in einem Schritt b) mittels eines Antriebes (SA) eine Verschiebebewegung auszuführen und hierdurch dieses Objekt (1) unter Entfernung desselben aus dem Startbereich (SB) auf der Unterlage (U) in den Erfassungsbereich (E) der Prüfeinrichtung (P) zu verschieben, so dass diese imstande ist, in einem Schritt c) den oder wenigstens einen der zu bestimmenden physikalischen oder chemischen Parameter dieses Objekts (1) zu erfassen,
- der Hub- und Senkmechanismus (HS) imstande ist, in einem Schritt d) einen lichten vertikalen Mindestabstand (D) zwischen Unterlage (U) und Verschiebeeinrichtung (S) herzustellen, welcher größer ist als die Vertikalausdehnung (d) des Objektes (1), wobei das Objekt (1) auf der Unterlage (U) verbleibt,
- die Verschiebeeinrichtung (S) imstande ist, anschließend in einem Schritt e) mittels des Antriebes (SA) eine der Verschiebebewegung entgegengesetzt gleiche Gegenbewegung auszuführen, ohne hierbei den Mindestabstand (D) zu unterschreiten und somit ohne das Objekt (1) zu verschieben oder zu berühren, und
- der Hub- und Senkmechanismus (HS) imstande ist, anschließend in einem Schritt f) den lichten vertikalen Abstand zwischen Verschiebeeinrichtung (S) und Unterlage (U) auf den Wert Null oder auf einen Wert, welcher kleiner ist als die Vertikalausdehnung des Objektes (1), zu vermindern und somit die Verschiebeeinrichtung (S) imstande ist, gegenüber der Unterlage (U) in die Anfangsstellung zurückzukehren, **dadurch gekennzeichnet, dass** der Antrieb ein Schwenkantrieb (SA) und die Verschiebebewegung sowie die Gegenbewegung jeweils Schwenkbewegungen sind.

2. Vorrichtung nach Anspruch 1, **dadurch gekennzeichnet, dass** die Verschiebeeinrichtung (S) imstande ist,
- im Schritt b) mittels des Schwenkantriebes (SA) als Verschiebebewegung in einer vorgegebenen Drehrichtung eine Vorwärts-Schwenkung von einer Anfangs- in eine Endstellung um einen vorgegebenen Vorwärts-Schwenkwinkel (ϕ) um eine vertikale oder zur Unterlage senkrechte Schwenkachse (A) auszuführen und hierdurch dieses Objekt (1) unter Entfernung desselben aus dem Startbereich (SB) auf der Unterlage (U) in den Erfassungsbereich (E) der Prüfeinrichtung (P) zu verschieben, so dass diese imstande ist, im Schritt c) den oder wenigstens einen der zu bestimmenden physikalischen oder chemischen Parameter dieses Objekts (1) zu erfassen,
- und im Schritt e) als Gegenbewegung mittels des Schwenkantriebes (SA) entgegen der vorgegebenen Drehrichtung eine Rückwärts-Schwenkung um einen dem Vorwärts-Schwenkwinkel (ϕ) entgegengesetzt gleichen Rückwärts-Schwenkwinkel (-ϕ) auszuführen, ohne hierbei den Mindestabstand (D) zu unterschreiten und somit ohne das Objekt (1) zu verschieben oder zu berühren.

3. Vorrichtung nach Anspruch 1 oder 2, **dadurch gekennzeichnet,**
**dass** die Verschiebeeinrichtung (S) imstande ist, im Schritt e) mittels des Schwenkantriebes (SA) anstelle der Gegenbewegung bzw. der Rückwärts-Schwenkung eine weitere Vorwärts-Schwenkung um einen solchen Winkel (360°-(ϕ) auszuführen, dass die Vorwärts-Schwenkung von Schritt b) und die weitere Vorwärts-Schwenkung sich zu einer vollen Drehung bzw. Schwenkung der Verschiebeeinrichtung (S) von insgesamt 360° ergänzen.

4. Vorrichtung nach einem der Ansprüche 1 bis 3, **dadurch gekennzeichnet,**
**dass** die Objekte (1,2,3,4,5) oder deren Überreste jeweils nach Ausführung des Schrittes c) aus dem Erfassungsbereich (E) der Prüfeinrichtung (P) entfernbar sind.

5. Vorrichtung nach einem der Ansprüche 1 bis 4, **dadurch gekennzeichnet,**
**dass** die Verschiebeeinrichtung (S) imstande ist, das Objekt (1,2,3,4,5) oder dessen Überreste nach Ausführung des Schrittes c) jeweils durch Wiederholung des Schrittes b) dem Erfassungsbereich (E',E") wenigstens einer weiteren Prüfeinrichtung (P',P") oder einem Zwischenlager, Zwischenbehälter (ZW) oder Sammelbehälter (B) zuzuführen.

6. Vorrichtung nach einem der Ansprüche 1 bis 5, **dadurch gekennzeichnet,**
**dass** die Verschiebeeinrichtung (S) wenigstens zwei vertikale, voneinander beabstandete Durchbrüche (B1,B2), insbesondere Durchgangsbohrungen (B1,B2), aufweist, welche je eines der Objekte (1,2,3,4,5) aufzunehmen imstande sind.

7. Vorrichtung nach Anspruch 6, **dadurch gekennzeichnet,**
**dass** die Zuführeinrichtung (Z) imstande ist, im Schritt a) eines der Objekte (1,2,3,4,5) in einen der Durchbrüche (B1) einzuführen, so dass sich dieses Objekt (1,2,3,4,5) während der Ausführung des Schrittes b) innerhalb dieses Durchbruches (B1) befindet.

8. Vorrichtung nach Anspruch 5 und 7, **dadurch gekennzeichnet,**
**dass** ein anderer der Durchbrüche (B2) so angeordnet ist, dass das Objekt (1,2,3,4,5) bei Ausführung des Schrittes f) in dem anderen Durchbruch (B2) aufgenommen wird, so dass das sich Objekt (1,2,3,4,5) bei erneuter Ausführung des Schrittes b) innerhalb dieses Durchbruches (B2) befindet.

9. Vorrichtung nach einem der vorigen Ansprüche, **dadurch gekennzeichnet,**
**dass** die Verschiebeeinrichtung (S) eine Schiebekante oder Schiebfläche (SF) aufweist, welche bei Vorwärts-Schwenkung der Verschiebeeinrichtung (S) eine Schubkraft auf das Objekt (1,2,3,4,5) ausübt und dieses hierdurch verschiebt,
wobei die Schiebekante oder Schiebfläche (SF) nicht radial zur Schwenkachse verläuft, so dass bei der Verschiebung des Objektes (1,2,3,4,5) durch die Schiebekante oder Schiebefläche (SF) reibungsbedingt eine Kraftkomponente nach innen in Richtung der Schwenkachse (A) oder nach außen in einer von der Schwenkachse (A) weggerichteten Richtung auf das Objekt (1,2,3,4,5) einwirkt.

10. Vorrichtung nach Anspruch 9, **dadurch gekennzeichnet,**
**dass** eine der Prüfeinrichtungen (P,P',P") einen Stempel aufweist, welcher zum Zweck der Bestimmung der horizontalen Ausdehnung und/oder der Härte des Objektes (1,2,3,4,5) parallel zur Unterlage (U) und parallel zur Schiebekante oder Schiebefläche (SF) verfahrbar ist.

11. Vorrichtung nach einem der vorigen Ansprüche, **dadurch gekennzeichnet,**
**dass** die Unterlage (U) teilweise durch einen Tisch (T) gebildet ist, auf welchen das Objekt (1,2,3,4,5) mittels der Verschiebeeinrichtung (S) aufschiebbar ist,
wobei der Tisch (T) gegenüber einem Anschlag (ANS) oder gegenüber einem Abstreifer (AS) in solcher Weise linear verfahrbar ist, dass das auf den Tisch (T) aufgeschobene Objekt (1,2,3,4,5) reibungsbedingt an einer bestimmten Position positioniert und in dem Fall, dass das Objekt (1,2,3,4,5) eine längliche Form aufweist, in einer bestimmten Richtung ausgerichtet wird.

12. Vorrichtung nach einem der vorigen Ansprüche, **dadurch gekennzeichnet,**
**dass**
- die Unterlage (U) teilweise durch einen Tisch (T) gebildet ist, auf welchen das Objekt (1,2,3,4,5) oder seine Überreste mittels der Verschiebeeinrichtung (S) aufschiebbar sind und unter welchem sich ein Hohlraum (H) befindet, und
- die Vorrichtung einen oberhalb des Tisches (T) angeordneten Abstreifer (AS) aufweist,
wobei der Tisch (T) gegenüber dem Abstreifer (AS) in solcher Weise linear verfahrbar ist, dass das Objekt (1,2,3,4,5) oder dessen Überreste vom Tisch (T) abgestreift werden und in den Hohlraum (H) fallen.

13. Vorrichtung nach einem der Ansprüche 9 bis 10 sowie nach Anspruch 11 oder 12, **dadurch gekennzeichnet,**
**dass** der Abstreifer (AS) durch die Schiebefläche (SF) gebildet ist.

14. Vorrichtung nach einem der vorigen Ansprüche, **gekennzeichnet durch** einen Aufprallsensor, welcher das Auftreffen eines Objekts (1,2,3,4,5) auf den Startbereich (SB) registriert und daraufhin die Ausführung des Schrittes b) auslöst.

15. Vorrichtung nach einem der vorigen Ansprüche, **dadurch gekennzeichnet,**
**dass** die Verschiebeeinrichtung (S) nach Ausführung des Schrittes b) und vor Ausführung des Schrittes d) oder vor Ausführung des Schrittes e) zunächst um einen bestimmten Zusatz-Vorwärts-Schwenkwinkel dϕ vorwärts schwenkbar und danach um einen dem Zusatz-Vorwärts-Schwenkwinkel dϕ entgegengesetzt gleichen Zusatz-Rückwärts-Schwenkwinkel -dϕ rückwärts schwenkbar ist.

16. Vorrichtung nach einem der vorigen Ansprüche, **dadurch gekennzeichnet,**
**dass** die Zuführeinrichtung (Z)
- ein Vorratsgefäß mit wenigstens einer Öffnung aufweist, in welchem die Objekte (1,2,3,4,5) vor Ausführung des Schritts a) angeordnet sind,
- und über dem Zwischenbehälter (ZW) angeordnet ist,
wobei die Zuführeinrichtung (Z) oder das Vorratsgefäß so schwenkbar sind, dass die Öffnung nach unten weist, wodurch die Objekte (1,2,3,4,5) direkt oder über eine Rutsche in den Zwischenbehälter (ZW) fallen.

17. Vorrichtung nach einem der vorigen Ansprüche außer Anspruch 3, **dadurch gekennzeichnet,**
**dass** die Verschiebeeinrichtung (S) anstelle der Schritte e) und f) mittels des Antriebes (SA) eine der Verschiebebewegung nicht entgegengesetzt gleiche Rückkehrbewegung ausführt, während welcher die Verschiebeeinrichtung (S) das Objekt (1) weder verschiebt noch berührt, und nach welcher sich die Verschiebeeinrichtung (S) wieder in der Ausgangsstellung befindet.

18. Verfahren zur Bestimmung wenigstens eines physikalischen oder chemischen Parameters, wie Gewicht, Härte, Dicke, Länge, Durchmesser, Löslichkeit, Schadstoffkonzentration, von Objekten (1,2,3,4,5) von fester oder gelartiger Konsistenz, insbesondere von pharmazeutischen Objekten, wie Tabletten, Pillen oder Oblongs (1,2,3,4,5), unter Verwendung einer Zuführeinrichtung (Z), einer Unterlage (U), einer Verschiebeeinrichtung (S), mindestens einer Prüfeinrichtung (P), wie Waage, Härteprüfer oder Längenmeßeinrichtung, sowie eines Hub- und Senkmechanismus (HS), welcher imstande ist, den vertikalen Abstand zwischen Verschiebeeinrichtung (S) und Unterlage zu verändern, wobei die Verschiebeeinrichtung (S) zunächst oberhalb der Unterlage (U) in einer Anfangsstellung angeordnet wird, in welcher der lichte vertikale Abstand zwischen Verschiebeeinrichtung (S) und Unterlage (U) kleiner ist als die Vertikalausdehnung des Objektes (1) oder den Wert Null aufweist, und danach folgende Schritte ausgeführt werden:
a) mittels der Zuführeinrichtung (Z) wird eines der Objekte (1) auf einen vorgegebenen Startbereich (SB) der Unterlage (U) geworfen, fallengelassen, aufgelegt oder in sonstige Weise lose auf der Unterlage (U) angeordnet,
b) die Verschiebeeinrichtung (S) wird mittels eines Antriebes (SA) einer Verschiebebewegung unterworfen hierdurch dieses Objekt (1) unter Entfernung desselben aus dem Startbereich (SB) auf der Unterlage (U) in den Erfassungsbereich (E) der Prüfeinrichtung (P) verschoben, so dass diese imstande ist, im Schritt c) den oder wenigstens einen der zu bestimmenden physikalischen oder chemischen Parameter dieses Objekts (1) zu erfassen,
c) der oder wenigstens einer der zu bestimmenden physikalischen oder chemischen Parameter dieses Objekts (1) werden mittels der Prüfeinrichtung (P) erfaßt,
d) mittels des Hub- und Senkmechanismus (HS) wird ein lichter vertikaler Mindestabstand (D) zwischen Unterlage (U) und Verschiebeeinrichtung (S) hergestelllt, welcher größer ist als die Vertikalausdehnung (d) des Objektes (1), wobei das Objekt (1) auf der Unterlage (U) verbleibt,
e) die Verschiebeeinrichtung (S) wird anschließend mittels des Antriebes (SA) einer der Verschiebebewegung entgegengesetzt gleichen Gegenbewegung unterworfen, wobei den Mindestabstand (D) nicht unterschritten und somit das Objekt (1) verschoben oder berührt wird, und
f) mittels des Hub- und Senkmechanismus (HS) wird anschließend der lichte vertikale Abstand zwischen Verschiebeeinrichtung (S) und Unterlage (U) auf den Wert Null oder auf einen Wert, welcher kleiner ist als die Vertikalausdehnung des Objektes (1), vermindert und somit die Verschiebeeinrichtung (S) gegenüber der Unterlage (U) in die Anfangsstellung zurückgebracht, **dadurch gekennzeichnet, dass** als Antrieb ein Schwenkantrieb (SA) verwendet wird und die Verschiebebewegung sowie die Gegenbewegung jeweils Schwenkbewegungen sind.

19. Verfahren nach Anspruch 18, **dadurch gekennzeichnet, dass** die Verschiebeeinrichtung (S)
- im Schritt b) mittels des Schwenkantriebes (SA) als Verschiebebewegung in einer vorgegebenen Drehrichtung einer Vorwärts-Schwenkung von einer Anfangs- in eine Endstellung um einen vorgegebenen Vorwärts-Schwenkwinkel (ϕ) um eine vertikale oder zur Unterlage senkrechte Schwenkachse (A) unterworfen wird und hierdurch dieses Objekt (1) unter Entfernung desselben aus dem Startbereich (SB) auf der Unterlage (U) in den Erfassungsbereich (E) der Prüfeinrichtung (P) verschoben wird, so dass diese imstande ist, in einem Schritt c) den oder wenigstens einen der zu bestimmenden physikalischen oder chemischen Parameter dieses Objekts (1) zu erfassen,
- und im Schritt e) mittels des Schwenkantriebes (SA) als Gegenbewegung entgegen der vorgegebenen Drehrichtung einer Rückwärts-Schwenkung um einen dem Vorwärts-Schwenkwinkel (ϕ) entgegengesetzt gleichen Rückwärts-Schwenkwinkel (-ϕ) unterzogen wird, wobei der Mindestabstand (D) nicht unterschritten und somit das Objekt (1)nicht verschoben oder berührt wird.

20. Verfahren nach Anspruch 18 oder 19, **dadurch gekennzeichnet,**
**dass** die Verschiebeeinrichtung (S) im Schritt e) mittels des Schwenkantriebes (SA) anstelle der Gegenbewegung bzw. der Rückwärts-Schwenkung einer weiteren Vorwärts-Schwenkung um einen solchen Winkel (360°-ϕ) unterzogen wird, dass die Vorwärts-Schwenkung von Schritt b) und die weitere Vorwärts-Schwenkung sich zu einer vollen Drehung bzw. Schwenkung der Verschiebeeinrichtung (S) von insgesamt 360° ergänzen.

21. Verfahren nach einem der Ansprüche 18 bis 20, **dadurch gekennzeichnet,**
**dass** die Objekte (1,2,3,4,5) oder deren Überreste jeweils nach Ausführung des Schrittes c) aus dem Erfassungsbereich (E) der Prüfeinrichtung (P) entfernt werden.

22. Verfahren nach einem der Ansprüche 18 bis 21, **dadurch gekennzeichnet,**
**dass** das Objekt (1,2,3,4,5) oder dessen Überreste nach Ausführung des Schrittes c) jeweils durch Wiederholung des Schrittes b) mittels der Verschiebeeinrichtung (S) dem Erfassungsbereich (E',E") wenigstens einer weiteren Prüfeinrichtung (P',P") oder einem Zwischenlager, Zwischenbehälter (ZW) oder Sammelbehälter (B) zugeführt werden.

23. Verfahren nach einem der Ansprüche 18 bis 22, **dadurch gekennzeichnet,**
**dass** die Verschiebeeinrichtung (S) wenigstens zwei vertikale voneinander beabstandete Durchbrüche (B1,B2), insbesondere Durchgangsbohrungen (B1,B2), aufweist, in welche je ein Objekt (1,2,3,4,5) aufgenommen wird.

24. Verfahren nach Anspruch 23, **dadurch gekennzeichnet,**
**dass** im Schritt a) ein Objekt (1,2,3,4,5) mittels der Zuführeinrichtung (Z) in einen der Durchbrüche (B1) eingeführt wird, so dass sich das Objekt (1,2,3,4,5) während der Ausführung des Schrittes b) innerhalb dieses Durchbruches (B1) befindet.

25. Verfahren nach Anspruch 22 und 24, **dadurch gekennzeichnet,**
**dass** das Objekt (1,2,3,4,5) durch Ausführung des Schrittes f) in einem anderen der Durchbrüche (B2) aufgenommen wird, so dass sich das Objekt (1,2,3,4,5) bei erneuter Ausführung des Schrittes b) innerhalb dieses Durchbruches (B2) befindet.

26. Verfahren nach einem der Ansprüche 18 bis 25, **dadurch gekennzeichnet,**
**dass** die Verschiebeeinrichtung (S) eine Schiebekante oder Schiebfläche (SF) aufweist, mittels welcher das Objekt (1,2,3,4,5) bei Vorwärts-Schwenkung der Verschiebeeinrichtung (S) verschoben wird, wobei als Verschiebeeinrichtung (S) eine solche verwendet wird, deren Schiebekante oder Schiebfläche (SF) nicht radial zur Schwenkachse verläuft, so dass bei der Verschiebung des Objektes (1,2,3,4,5) durch die Schiebekante oder Schiebefläche (SF) reibungsbedingt eine Kraftkomponente nach innen in Richtung der Schwenkachse (A) oder nach außen in eine von der Schwenkachse (A) weggerichteten Richtung auf das Objekt (1,2,3,4,5) ausgeübt wird.

27. Verfahren nach einem der Ansprüche 18 bis 26, **dadurch gekennzeichnet,**
**dass** als Unterlage (U) eine solche verwendet wird, welche teilweise durch einen Tisch (T) gebildet ist, auf welchen das Objekt (1,2,3,4,5) mittels der Verschiebeeinrichtung (S) aufgeschoben wird, wobei der Tisch (T) gegenüber einem Anschlag (ANS) oder gegenüber einem Abstreifer (AS) in solcher Weise linear verfahren wird, dass das auf den Tisch (T) aufgeschobene Objekt (1,2,3,4,5) reibungsbedingt an einer bestimmten Position positioniert und in dem Fall, dass das Objekt (1,2,3,4,5) eine längliche Form aufweist, in einer bestimmten Richtung ausgerichtet wird.

28. Verfahren nach einem der Ansprüche 18 bis 27, **dadurch gekennzeichnet,**
**dass**
- als Unterlage (U) eine solche verwendet wird, welche teilweise durch einen Tisch (T) gebildet ist, auf welchen das Objekt (1,2,3,4,5) oder seine Überreste mittels der Verschiebeeinrichtung (S) aufgeschoben werden und unter welchem sich ein Hohlraum (H) befindet, und
- ein oberhalb des Tisches (T) angeordneten Abstreifer (AS) verwendet wird,
wobei der Tisch (T) gegenüber dem Abstreifer (AS) in solcher Weise horizontal verfahren wird, dass das Objekt (1,2,3,4,5) oder dessen Überreste vom Tisch (T) abgestreift werden und in den Hohlraum (H) fallen.

29. Verfahren nach einem der Ansprüche 26 bis 27 sowie nach Anspruch 28,
**dadurch gekennzeichnet,**
**dass** die Schiebefläche (SF) als Abstreifer (AS) verwendet wird.

30. Verfahren nach einem der Ansprüche 18 bis 29, **dadurch gekennzeichnet,**
**dass** die Verschiebeeinrichtung (S) nach Ausführung des Schrittes b) und vor Ausführung des Schrittes d) oder vor Ausführung des Schrittes e) zunächst um einen bestimmten Zusatz-Vorwärts-Schwenkwinkel dϕ vorwärts geschwenkt und danach um einen dem Zusatz-Vorwärts-Schwenkwinkel dϕ entgegengesetzt gleichen Zusatz-Rückwärts-Schwenkwinkel -dϕ rückwärts geschwenkt wird.

31. Verfahren nach einem der Ansprüche 18 bis 30, **dadurch gekennzeichnet,**
**dass** anstelle der Schritte e) und f) folgender Schritt ausgeführt wird: die Verschiebeeinrichtung (S) wird mittels des Antriebes (SA) einer der Verschiebebewegung nicht entgegengesetzt gleichen Rückkehrbewegung unterworfen, durch welche die Verschiebeeinrichtung (S) wieder in die Ausgangsstellung gebracht wird, ohne das Objekt (1) zu verschieben oder zu berühren.

## Claims

1. A device for determining at least one physical or chemical parameter such as the weight, hardness, thickness, length, diameter, solubility or contaminant concentration of objects (1, 2, 3, 4, 5) having a solid or gel-like consistency, especially of pharmaceutical objects such as tablets, pills or oblongs (1, 2, 3, 4, 5), comprising a feed device (Z), a substrate (U), a displacement device (S), at least one testing device (P) such as a set of scales, hardness tester or length measuring device, as well as a lifting and lowering mechanism (HS), which is capable of changing the vertical distance between the displacement device (S) and the substrate, whereby
- in a Step a), the feed device (Z) is capable of throwing, dropping or laying one of the objects (1) onto a predefined starting area (SB) of the substrate (U) or otherwise arranging one of the objects (1) loosely on the substrate (U), whereby the displacement device (S) is arranged above the substrate (U) in an initial position in which the free vertical distance between the displacement device (S) and the substrate (U) is smaller than the vertical extension of the object (1) or else it has a value of zero,
- in a Step b), the displacement device (S) is capable of executing a displacement motion by means of a drive (SA), thereby removing this object (1) from the starting area (SB) on the substrate (U), and displacing it into the detection area (E) of the testing device (P) so that, in a Step c), said testing device (P) is capable of detecting the or at least one of the physical or chemical parameters that are to be determined for this object (1),
- in a Step d), the lifting and lowering mechanism (HS) is capable of creating a minimum free vertical distance (D) between the substrate (U) and the displacement device (S) that is greater than the vertical extension (d) of the object (1), whereby the object (1) remains on the substrate (U),
- subsequently, in a Step e), by means of the drive (SA), the displacement device (S) is capable of executing an equivalent counter-motion that is opposite to the displacement motion, without falling below the minimum distance (D) and thus without displacing or touching the object (1),
- subsequently, in a Step f), the lifting and lowering mechanism (HS) is capable of reducing the free vertical distance between the displacement device (S) and the substrate (U) to a value of zero or to a value that is smaller than the vertical extension of the object (1), and thus the displacement device (S) is capable of returning to the initial position relative to the substrate (U),
**characterized in that** the drive is a pivoting drive (SA) and the displacement motion as well as the counter-motion are each pivoting motions.

2. The device according to Claim 1, **characterized in that** the displacement device (S) is capable,
- in Step b), by means of the pivoting drive (SA), of executing a forward pivoting motion as a displacement motion in a predefined direction of rotation from an initial position to a final position by a predefined forward pivoting angle (ϕ) around a pivoting axis (A) that is vertical or else that is perpendicular to the substrate, and in this manner, of removing this object (1) from the starting area (SB) on the substrate (U), and of displacing it into the detection area (E) of the testing device (P) so that, in a Step c), said testing device (P) is capable of detecting the or at least one of the physical or chemical parameters that are to be determined for this object (1),
- and in Step e), by means of the pivoting drive (SA), of executing a backward pivoting motion around an equivalent backward pivoting angle (-ϕ) that is opposite to the forward pivoting angle (ϕ), as a counter-motion without falling below the minimum distance (D) and thus without displacing or touching the object (1).

3. The device according to Claim 1 or 2, **characterized in that** in Step e), by means of the pivoting drive (SA), the displacement device (S), instead of the counter-motion or the backward pivoting motion is capable of executing an additional forward pivoting motion by such an angle (360°-ϕ) that the forward pivoting motion of Step b) and the additional forward pivoting motion complement each other to create a full rotation or pivoting motion of the displacement device (S) totaling 360°.

4. The device according to any of Claims 1 to 3, **characterized in that** after Step c) has been carried out, the objects (1, 2, 3, 4, 5) or their remnants can be removed from the detection area (E) of the testing device (P).

5. The device according to any of Claims 1 to 4, **characterized in that** after Step c) has been carried out and by repeating Step b), the displacement device (S) is capable of feeding the object (1, 2, 3, 4, 5) or its remnants to the detection area (E', E") of at least one additional testing device (P', P") or to a buffer, intermediate container (ZW) or collecting container (B).

6. The device according to any of Claims 1 to 5, **characterized in that** the displacement device (S) has at least two vertical openings (B1, B2), especially through holes (B1, B2), that are at a distance from each other and that are each capable of receiving one of the objects (1, 2, 3, 4, 5).

7. The device according to Claim 6, **characterized in that** in Step a), the feed device (Z) is capable of inserting one of the objects (1, 2, 3, 4, 5) into one of the openings (B1) so that this object (1, 2, 3, 4, 5) is located inside this opening (B1) during the execution of Step b).

8. The device according to Claims 5 and 7, **characterized in that** another one of the openings (B2) is arranged in such a way that, when Step f) is carried out, the object (1, 2, 3, 4, 5) is received in the other opening (B2), so that the object (1, 2, 3, 4, 5) is located inside this opening (B2) when Step b) is carried out once again.

9. The device according to any of the preceding claims, **characterized in that**
the displacement device (S) has a pushing edge or pushing surface (SF) that, during the forward pivoting motion of the displacement device (S), exerts a pushing force on the object (1, 2, 3, 4, 5), thus displacing it, whereby the pushing edge or pushing surface (SF) does not run radially to the pivoting axis, so that, when the object (1, 2, 3, 4, 5) is being displaced by the pushing edge or pushing surface (SF), friction causes a force component to act on the object (1, 2, 3, 4, 5) towards the inside in the direction of the pivoting axis (A) or towards the outside in a direction facing away from the pivoting axis (A).

10. The device according to Claim 9, **characterized in that**
one of the testing devices (P, P', P") has a stamp which, in order for the horizontal extension and/or the hardness of the object (1, 2, 3, 4, 5) to be determined, can be moved parallel to the substrate (U) and parallel to the pushing edge or pushing surface (SF).

11. The device according to any of the preceding claims, **characterized in that** the substrate (U) is partially formed by a table (T) onto which the object (1, 2, 3, 4, 5) can be pushed by means of the displacement device (S), whereby the table (T) can be moved linearly relative to a stop (ANS) or relative to a scraper (AS) in such a way that friction causes the object (1, 2, 3, 4, 5) that has been pushed onto the table (T) to be positioned in a certain position and, in case the object (1, 2, 3, 4, 5) has an elongated shape, to be aligned in a certain direction.

12. The device according to any of the preceding claims, **characterized in that**
- the substrate (U) is partially formed by a table (T) onto which the object (1, 2, 3, 4, 5) or its remnants can be pushed by means of the displacement device (S) and below which there is a cavity (H), and
- the device has a scraper (AS) arranged above the table (T),
whereby the table (T) can be moved linearly relative to the scraper (AS) in such a way that the object (1, 2, 3, 4, 5) or its remnants are scraped off the table (T) and drop into the cavity (H).

13. The device according to either Claim 9 or 10, as well as according to Claim 11 or 12, **characterized in that**
the scraper (AS) is formed by the pushing surface (SF).

14. The device according to any of the preceding claims, **characterized by**
an impact sensor that detects when an object (1, 2, 3, 4, 5) reaches the starting area (SB) and then triggers the execution of Step b).

15. The device according to any of the preceding claims, **characterized in that**
after Step b) has been carried out and before Step d) is carried out or before Step e) is carried out, the displacement device (S) can first be pivoted forward by a certain additional forward pivoting angle dϕ and can subsequently be pivoted backward by an equivalent additional backward pivoting angle -dϕ that is opposite to the additional forward pivoting angle dϕ.

16. The device according to any of the preceding claims, **characterized in that**
the feed device (Z)
- has a reservoir with at least one opening in which the objects (1, 2, 3, 4, 5) are arranged before Step a) is carried out,
- and it is arranged above the intermediate container (ZW),
whereby the feed device (Z) or the reservoir can be pivoted in such a manner that the opening faces downward, as a result of which the objects (1, 2, 3, 4, 5) drop directly or via a chute into the intermediate container (ZW).

17. The device according to any of the preceding claims except for Claim 3, **characterized in that**,
instead of Steps e) and f), by means of the drive (SA), the displacement device (S) executes an equivalent return motion that is not opposite to the displacement motion, during which the displacement device (S) neither pushes nor touches the object (1), and after which the displacement device (S) is once again in its initial position.

18. A method for determining at least one physical or chemical parameter such as the weight, hardness, thickness, length, diameter, solubility or contaminant concentration of objects (1, 2, 3, 4, 5) having a solid or gel-like consistency, especially of pharmaceutical objects such as tablets, pills or oblongs (1, 2, 3, 4, 5), making use of a feed device (Z), a substrate (U), a displacement device (S), at least one testing device (P) such as a set of scales, hardness tester or length measuring device, as well as a lifting and lowering mechanism (HS), which is capable of changing the vertical distance between the displacement device (S) and the substrate (U), whereby the displacement device (S) is first arranged above the substrate (U) in an initial position in which the free vertical distance between the displacement device (S) and the substrate (U) is smaller than the vertical extension of the object (1) or else it has a value of zero, after which the following steps are carried out:
a) by means of the feed device (Z), one of the objects (1) is thrown, dropped or laid onto a predefined starting area (SB) of the substrate (U) or otherwise arranged loosely on the substrate (U),
b) by means of a drive (SA), the displacement device (S) undergoes a displacement motion, thereby removing this object (1) from the starting area (SB) on the substrate (U), and displacing it into the detection area (E) of the testing device (P) so that, in a Step c), said testing device (P) is capable of detecting the or at least one of the physical or chemical parameters that are to be determined for this object (1),
c) the or at least one of the physical or chemical parameters that are to be determined for this object (1) are detected by means of the testing device (P),
d) by means of the lifting and lowering mechanism (HS), a minimum free vertical distance (D) between the substrate (U) and the displacement device (S) is created that is greater than the vertical extension (d) of the object (1), whereby the object (1) remains on the substrate (U),
e) by means of the drive (SA), the displacement device (S) subsequently undergoes an equivalent counter-motion that is opposite to the displacement motion, without falling below the minimum distance (D) and thus displacing or touching the object (1), and
f) subsequently, by means of the lifting and lowering mechanism (HS), the free vertical distance between the displacement device (S) and the substrate (U) is reduced to a value of zero or to a value that is smaller than the vertical extension of the object (1), and thus the displacement device (S) is returned to the initial position relative to the substrate (U),
**characterized in that** a pivoting drive (SA) is used as the drive and the displacement motion as well as the counter-motion are each pivoting motions.

19. The method according to Claim 18, **characterized in that**
- in Step b), by means of the pivoting drive (SA), the displacement device (S) undergoes a forward pivoting motion as a displacement motion in a predefined direction of rotation from an initial position to a final position by a predefined forward pivoting angle (ϕ) around a pivoting axis (A) that is vertical or else that is perpendicular to the substrate, thereby removing this object (1) from the starting area (SB) on the substrate (U), and displacing it into the detection area (E) of the testing device (P) so that, in a Step c), said testing device (P) is capable of detecting the or at least one of the physical or chemical parameters that are to be determined for this object (1),
- and in Step e), by means of the pivoting drive (SA), as a counter-motion opposite to the predefined direction of rotation, the displacement device (S) undergoes a backward pivoting motion by an equivalent backward pivoting angle (-ϕ) that is opposite to the forward pivoting angle (ϕ), without falling below the minimum distance (D), and thus the object (1) is not displaced or touched.

20. The method according to Claim 18 or 19, **characterized in that**, in Step e), instead of the counter-motion or the backward pivoting motion, by means of the pivoting drive (SA), the displacement device (S) undergoes an additional forward pivoting motion by such an angle (360°-ϕ) that the forward pivoting motion of Step b) and the additional forward pivoting motion complement each other to create a full rotation or pivoting motion of the displacement device (S) totaling 360°.

21. The method according to any of Claims 18 to 20, **characterized in that**
after Step c) has been carried out, the objects (1, 2, 3, 4, 5) or their remnants are removed from the detection area (E) of the testing device (P).

22. The method according to any of Claims 18 to 21, **characterized in that**
after Step c) has been carried out and by repeating Step b), the object (1, 2, 3, 4, 5) or its remnants are fed by means of the displacement device (S) to the detection area (E', E") of at least one additional testing device (P', P") or to a buffer, intermediate container (ZW) or collecting container (B).

23. The method according to any of Claims 18 to 22, **characterized in that**
the displacement device (S) has at least two vertical openings (B1, B2), especially through holes (B1, B2), that are at a distance from each other, in each of which one of the objects (1, 2, 3, 4, 5) is received.

24. The method according to Claim 23, **characterized in that**,
in Step a), the feed device (Z) inserts an object (1, 2, 3, 4, 5) into one of the openings (B1) so that the object (1, 2, 3, 4, 5) is located inside this opening (B1) during the execution of Step b).

25. The method according to Claims 22 and 24, **characterized in that**, when Step f) is carried out, the object (1, 2, 3, 4, 5) is received in another one of the openings (B2), so that the object (1, 2, 3, 4, 5) is located inside this opening (B2) when Step b) is carried out once again.

26. The method according to any of Claims 18 to 25, **characterized in that**
the displacement device (S) has a pushing edge or pushing surface (SF) by means of which the object (1, 2, 3, 4, 5) is displaced during the forward pivoting motion of the displacement device (S), whereby the displacement device (S) used is one whose pushing edge or pushing surface (SF) does not run radially to the pivoting axis, so that, when the object (1, 2, 3, 4, 5) is being displaced by the pushing edge or pushing surface (SF), friction causes a force component to be exerted on the object (1, 2, 3, 4, 5) towards the inside in the direction of the pivoting axis (A) or towards the outside in a direction facing away from the pivoting axis (A).

27. The method according to any of Claims 18 to 26, **characterized in that**,
the substrate (U) used is one that is partially formed by a table (T) onto which the object (1, 2, 3, 4, 5) is pushed by means of the displacement device (S), whereby the table (T) is moved linearly relative to a stop (ANS) or relative to a scraper (AS) in such a way that friction causes the object (1, 2, 3, 4, 5) that has been pushed onto the table to be positioned in a certain position and, in case the object (1, 2, 3, 4, 5) has an elongated shape, to be aligned in a certain direction.

28. The method according to any of Claims 18 to 27, **characterized in that**
- the substrate (U) used is one that is partially formed by a table (T) onto which the object (1, 2, 3, 4, 5) or its remnants are pushed by means of the displacement device (S) and below which there is a cavity (H), and
- a scraper (AS) arranged above the table (T) is used,
whereby the table (T) is moved horizontally relative to the scraper (AS) in such a way that the object (1, 2, 3, 4, 5) or its remnants are scraped off the table (T) and drop into the cavity (H).

29. The method according to either Claim 26 or 27 as well as Claim 28,
**characterized in that**
the pushing surface (SF) is used as a scraper (AS) .

30. The method according to any of Claims 18 to 29, **characterized in that**
after Step b) has been carried out and before Step d) is carried out or before Step e) is carried out, the displacement device (S) is first pivoted forward by a certain additional forward pivoting angle dϕ and is subsequently pivoted backward by an equivalent additional backward pivoting angle -dϕ that is opposite to the additional forward pivoting angle dϕ.

31. The method according to any of Claims 18 to 30, **characterized in that**
instead of Steps e) and f), the following steps are carried out: by means of the drive (SA), the displacement device (S) undergoes an equivalent return motion that is not opposite to the displacement motion and by means of which the displacement device (S) is moved back into the initial position without displacing or touching the object (1).

## Revendications

1. Dispositif de détermination d'au moins un paramètre physique ou chimique, tel que le poids, la dureté, l'épaisseur, la longueur, le diamètre, la solubilité, la concentration en substances nocives d'objets (1,2,3,4,5) de consistance solide ou de consistance de gélule, notamment d'objets pharmaceutiques, tels que des comprimés, des pilules ou des oblongs (1,2,3,4,5), comprenant un dispositif d'amenée (Z), un support (U), un dispositif de déplacement (S), au moins un dispositif de contrôle (P) tel qu'une balance, un appareil pour essais de dureté, ou un dispositif pour mesure de la longueur, ainsi qu'un mécanisme de levage et d'abaissement (HS), lequel est en mesure de modifier l'écart vertical entre le dispositif de déplacement (S) et le support, dans lequel
- le dispositif d'amenée (Z) est en mesure, dans une étape a), de jeter, de faire tomber, de poser ou de disposer individuellement de quelque autre façon l'un des objets (1) sur une zone de départ définie (SB) du support (U), le dispositif de déplacement (S) étant disposé au-dessus du support (U) dans une position de départ, dans laquelle l'écartement vertical entre le dispositif de déplacement (S) et le support (U) est plus petit que la dimension verticale de l'objet (1) ou présente la valeur zéro,
- le dispositif de déplacement (S) est en mesure, dans une étape b), d'effectuer, au moyen d'un entraînement (SA), un mouvement de déplacement et de déplacer de par-là même cet objet (1) en l'éloignant en dehors de la zone de départ (SB) sur le support (U) jusque dans la zone de saisie (E) du dispositif de contrôle (P), de sorte que celui-ci est en mesure, dans une étape c), de saisir le paramètre ou tout au moins l'un des paramètres physique ou chimique à déterminer pour cet objet (1),
- le mécanisme de levage et d'abaissement (HS) est en mesure, dans une étape d), de réaliser un écart minima vertical (D) entre le support (U) et le dispositif de déplacement (S), lequel écart est plus grand que la dimension verticale (d) de l'objet (1), l'objet (1) demeurant sur le support (U),
- le dispositif de déplacement (S) est en mesure d'effectuer, par la suite dans une étape e) au moyen de l'entraînement (SA), un même mouvement à contrario opposé au mouvement de déplacement, sans se trouver ce faisant en deça de l'écart minima (D) et de ce fait, sans déplacer ni toucher l'objet (1), et
- le mécanisme de levage et d'abaissement (HS) est en mesure, par la suite dans une étape f), de réduire l'écart vertical entre le dispositif de déplacement (S) et le support (U) à la valeur zéro ou à une valeur plus petite que la dimension verticale de l'objet (1) et de ce fait, le dispositif de déplacement (S) est en mesure de retourner dans la position de départ par rapport au support (U),
**caractérisé en ce que** l'entraînement est un entraînement pivotant (SA) et le mouvement de déplacement, ainsi que le mouvement à contrario, sont respectivement des mouvements de pivotement.

2. Dispositif selon la revendication 1, **caractérisé en ce que**
le dispositif de déplacement (S) est en mesure,
- dans l'étape b), d'effectuer au moyen de l'entraînement pivotant (SA), un pivotement en avant dans un sens de rotation prédéfini, en tant que mouvement de déplacement depuis une position de départ jusqu'à une position finale, d'un angle de pivotement en avant (ϕ) prédéfini, autour d'un axe de pivotement (A) vertical ou vertical au support, et de déplacer de par-là même cet objet (1) en l'éloignant en dehors de la zone de départ (SB) sur le support (U) jusque dans la zone de saisie (E) du dispositif de contrôle (P), de sorte que celui-ci est en mesure, dans une étape c) de saisir le paramètre ou tout du moins l'un des paramètres physique ou chimique à déterminer pour cet objet (1),
- et, dans l'étape e), d'effectuer comme mouvement à contrario au moyen de l'entraînement pivotant (SA) un pivotement en arrière à l'opposé du sens de pivotement prédéfini, d'un même angle de pivotement en arrière (-ϕ) opposé à l'angle de pivotement en avant (ϕ), sans se trouver ce faisant en deça de l'écart minima (D) et de ce fait, sans déplacer ni toucher l'objet (1).

3. Dispositif selon la revendication 1 ou 2, **caractérisé en ce que**
le dispositif de déplacement (S) est en mesure, dans l'étape e) d'effectuer, au moyen de l'entraînement pivotant (SA), au lieu du mouvement à contrario ou du pivotement en arrière, un nouveau pivotement en avant d'un angle (-ϕ 360°) tel que le pivotement en avant de l'étape b) et le nouveau pivotement en avant se complètent pour effectuer un tour ou un pivotement complet du dispositif de déplacement (S) de 360° en tout.

4. Dispositif selon l'une des revendications 1 à 3, **caractérisé en ce que**
les objets (1,2,3,4,5) ou leurs débris peuvent être enlevés de la zone de saisie (E) du dispositif de contrôle (P) après chaque exécution de l'étape c).

5. Dispositif selon l'une des revendications 1 à 4, **caractérisé en ce que**
le dispositif de déplacement (S) est en mesure, après l'exécution de l'étape c), d'amener, en répétant à chaque fois l'étape b), l'objet (1,2,3,4,5) ou ses débris dans la zone de saisie (E', E"), tout au moins à un autre dispositif de contrôle (P', P") ou à un stockage intermédiaire, un contenant intermédiaire (ZW) ou à un récipient collecteur (B).

6. Dispositif selon l'une des revendications 1 à 5, **caractérisé en ce que**
le dispositif de déplacement (S) présente au moins deux percées (B1,B2) verticales espacées l'une de l'autre, notamment des trous de passage (B1,B2), lesquels sont en mesure de recevoir chacun l'un des objets (1,2,3,4,5).

7. Dispositif selon la revendication 6, **caractérisé en ce que**
le dispositif d'amenée (Z) est en mesure, dans l'étape a), d'introduire l'un des objets (1,2,3,4,5) dans l'une des percées (B1,B2), de sorte que cet objet (1,2,3,4,5) se trouve à l'intérieur de cette percée (B1) pendant l'exécution de l'étape b).

8. Dispositif selon la revendication 5 et 7, **caractérisé en ce que**
une autre des percées (B2) est disposée de sorte que l'objet (1,2,3,4,5) est receuilli lors de l'exécution de l'étape f) dans une autre percée (B2), de sorte que l'objet (1,2,3,4,5) se trouve, lors d'une nouvelle exécution de l'étape b), à l'intérieur de cette percée (B2).

9. Dispositif selon l'une des revendications précédentes, **caractérisé en ce que**
le dispositif de déplacement (S) présente une arête de poussée ou surface de poussée (SF) qui exerce, lors du pivotement en avant du dispositif de déplacement (S), une poussée sur l'objet (1,2,3,4,5) et déplace de ce fait celui-ci, l'arête de poussée ou surface de poussée (SF) n'ayant pas un tracé radial par rapport à l'axe de pivotement, de sorte que lors du déplacement de l'objet (1,2,3,4,5) par l'arête de poussée ou surface de poussée (SF), une composante de force due au frottement agit sur l'objet (1,2,3,4,5) vers l'intérieur en direction de l'axe de pivotement (A) ou vers l'extérieur, dans une direction opposée à l'axe de pivotement (A).

10. Dispositif selon la revendication 9, **caractérisé en ce que**
l'un des dispositifs de contrôle (P,P',P") présente un pilon qui est déplaçable parallèlement au support (U) et parallèlement à l'arête de poussée, dans le but de mesurer la dimension horizontale et/ou la dureté de l'objet (1,2,3,4,5).

11. Dispositif selon l'une des revendications précédentes, **caractérisé en ce que** le support (U) est formé partiellement par une table (T) sur laquelle l'objet (1,2,3,4,5) peut être poussé au moyen du dispositif de déplacement (S), la table (T) étant déplaçable contre une butée (ANS) ou contre une racle (AS) dans un sens linéaire, de manière à ce que l'objet (1,2,3,4,5) poussé sur la table (T) se positionne en raison du frottement dans une position définie et, au cas où l'objet (1,2,3,4,5) présente une forme oblongue, il s'oriente dans un sens défini.

12. Dispositif selon l'une des revendications précédentes, **caractérisé en ce que**
- le support (U) est formé partiellement par une table (T) sur laquelle l'objet (1,2,3,4,5) ou ses débris peuvent être poussés au moyen du dispositif de déplacement (S) et au-dessous de laquelle se trouve un espace vide (H), et
- le dispositif présente une racle (AS) disposée au-dessus de la table (T), la table (T) étant déplaçable par rapport à la racle (AS) dans un sens linéaire, de sorte que l'objet (1,2,3,4,5) ou ses débris sont raclés de la table (T) et tombent dans l'espace vide (H).

13. Dispositif selon l'une des revendications 9 à 10, ainsi que selon la revendication 11 ou 12, **caractérisé en ce que**,
la racle (AS) est formée par la surface de poussée (SF).

14. Dispositif selon l'une des revendications précédentes, **caractérisé par**
un capteur d'impact qui enregistre l'impact d'un objet (1,2,3,4,5) sur la zone de départ (SB) et déclenche suite à cela l'exécution de l'étape b).

15. Dispositif selon l'une des revendications précédentes, **caractérisé en ce que** le dispositif de déplacement (S) peut, après l'exécution de l'étape b) et avant l'exécution de l'étape d) ou avant l'exécution de l'étape e), d'abord pivoter en avant d'un angle de pivotement en avant supplémentaire défini dϕ et pivoter ensuite en arrière d'un même angle de pivotement en arrière supplémentaire - dϕ opposé à l'angle de pivotement en avant supplémentaire dϕ.

16. Dispositif selon l'une des revendications précédentes, **caractérisé en ce que** le dispositif d'amenée (Z)
- présente un contenant de réserve avec au moins une ouverture, dans lequel les objets (1,2,3,4,5) sont disposés avant l'exécution de l'étape a),
- et est disposé au-dessus du contenant intermédiaire (ZW), le dispositif d'amenée (Z) ou le contenant de réserve pouvant pivoter de sorte que l'ouverture se présente vers le bas, ce par quoi les objets (1,2,3,4,5) tombent dans le contenant intermédiaire directement ou par le biais d'un glissoir.

17. Dispositif selon l'une des revendications précédentes, sauf la revendication 3, **caractérisé en ce que**
le dispositif de déplacement (S) effectue au lieu des étapes e) et f), au moyen de l'entraînement (SA), un même mouvement de retour non contraire au mouvement de déplacement, mouvement pendant lequel le dispositif de déplacement ne déplace ni ne touche l'objet (1) et après lequel le dispositif de déplacement (S) se trouve à nouveau dans la position initiale.

18. Procédé de détermination d'au moins un paramètre physique ou chimique, tel que le poids, la dureté, l'épaisseur, la longueur, le diamètre, la solubilité, la concentration en substances nocives d'objets (1,2,3,4,5) de consistance solide ou de consistance de gélule, notamment d'objets pharmaceutiques, tels que des comprimés, des pilules ou des oblongs (1,2,3,4,5), ayant recours à un dispositif d'amenée (Z), à un support (U), à un dispositif de déplacement (S), à au moins un dispositif de contrôle (P) tel qu'une balance, un appareil pour essais de dureté, ou à un dispositif pour mesure de la longueur, ainsi qu'à un mécanisme de levage et d'abaissement (HS), lequel est en mesure de modifier l'écart vertical entre le dispositif de déplacement (S) et le support (U), le dispositif de déplacement (S) étant disposé d'abord au-dessus du support (U) dans une position initiale dans laquelle l'écart vertical entre le dispositif de déplacement (S) et le support (U) est plus petit que la dimension verticale de l'objet (1) ou présente la valeur zéro, et ensuite, les étapes suivantes sont effectuées :
a) au moyen du dispositif d'amenée (Z), l'un des objets (1) est jeté, tombe, est posé, déposé sur une zone de départ (SB) définie du support (U), ou disposé individuellement de quelque autre façon sur le support (U),
b) le dispositif de déplacement (S) est soumis à un mouvement de déplacement au moyen d'un entraînement (SA), ce par quoi cet objet (1) est poussé sur le support (U) en s'éloignant en dehors de la zone de départ (SB) jusqu'à la zone de saisie (E) du dispositif de contrôle (P), de sorte que celui-ci est en mesure, dans l'étape c), de saisir le paramètre ou tout au moins l'un des paramètres physique ou chimique à déterminer pour cet objet (1),
c) le paramètre ou tout du moins l'un des paramètres physique ou chimique à déterminer pour cet objet (1) sont saisis au moyen du dispositif de contrôle (P),
d) au moyen du mécanisme de levage et d'abaissement (HS), un écart vertical minima (D) est réalisé entre le support (U) et le dispositif de déplacement (S), lequel écart est plus grand que la dimension verticale de l'objet (1), l'objet (1) demeurant sur le support (U),
e) le dispositif de déplacement (S) est ensuite soumis au moyen de l'entraînement (SA) à un même mouvement à contrario opposé au mouvement de déplacement, sans se trouver en deça de l'écart minima (D), et de ce fait, sans déplacer ni toucher l'objet (1), et
f) au moyen du mécanisme de levage et d'abaissement (HS), l'écart vertical entre le dispositif de déplacement (S) et le support (U) est ensuite réduit à la valeur zéro ou à une valeur qui est plus petite que la dimension verticale de l'objet (1), et le dispositif de déplacement (S) retourne ainsi dans sa position de départ par rapport au support (U),
**caractérisé en ce que** l'entraînement utilisé est un entraînement pivotant (SA) et le mouvement de déplacement, ainsi que le mouvement à contrario sont des mouvements pivotants.

19. Procédé selon la revendication 18, **caractérisé en ce que**
le dispositif de déplacement (S)
- est soumis, dans l'étape b), au moyen de l'entraînement pivotant (SA), à un pivotement en avant dans un sens de rotation prédéfini en tant que mouvement de déplacement, depuis une position de départ jusqu'à une position finale, d'un angle de pivotement en avant (ϕ) prédéfini autour d'un axe de pivotement (A) vertical ou vertical au support, et, de par-là même, cet objet (1) se déplace en s'éloignant en dehors de la zone de départ (SB) sur le support (U) jusque dans la zone de saisie (E) du dispositif de contrôle (P), de sorte que celui-ci est en mesure, dans une étape c) de saisir le paramètre ou tout du moins l'un des paramètres physique ou chimique à déterminer pour cet objet (1),
- et, dans l'étape e), est soumis au moyen de l'entraînement pivotant (SA) à un pivotement en arrière en tant que mouvement à contrario à l'opposé du sens de rotation prédéfini, d'un même angle de pivotement en arrière (-ϕ) opposé à l'angle de pivotement en avant (ϕ), sans se trouver ce faisant en deça de l'écart minima (D), et de ce fait sans déplacer ni toucher l'objet (1).

20. Procédé selon la revendication 18 ou 19, **caractérisé en ce que**
le dispositif de déplacement (S) est soumis dans l'étape (e) au moyen de l'entraînement pivotant (SA), au lieu du mouvement à contrario ou du pivotement en arrière, à un autre pivotement en avant d'un angle (-ϕ 360°), de sorte que le pivotement en avant de l'étape b) et le nouveau pivotement en avant se complètent pour réaliser un tour ou un pivotement complet du dispositif de déplacement (S) de 360° en tout.

21. Procédé selon l'une des revendications 18 à 20, **caractérisé en ce que**
les objets (1,2,3,4,5) ou leurs débris sont enlevés de la zone de saisie (E) du dispositif de contrôle (P) après chaque exécution de l'étape c).

22. Procédé selon l'une des revendications 18 à 21, **caractérisé en ce que**
après l'exécution de l'étape c), l'objet (1,2,3,4,5) ou ses débris sont amenés à chaque fois par répétition de l'étape b), au moyen du dispositif de déplacement (S), dans la zone de saisie (E',E"), au moins à un autre dispositif de contrôle (P', P") ou à un stockage intermédiaire, un contenant intermédiaire (ZW) ou à un récipient collecteur (B).

23. Procédé selon l'une des revendications 18 à 22, **caractérisé en ce que**
le dispositif de déplacement (S) présente au moins deux percées (B1,B2) verticales (B1, B2) espacées l'une de l'autre, notamment deux trous de passage (B1,B2) lesquels recueillent chacun un objet (1,2,3,4,5).

24. Procédé selon la revendication 23, **caractérisé en ce que**
dans l'étape a), un objet (1,2,3,4,5) est introduit au moyen du dispositif d'amenée (Z) dans l'une des percées (B1), de sorte que l'objet (1,2,3,4,5) se trouve à l'intérieur de cette percée (B1) pendant l'exécution de l'étape b).

25. Procédé selon les revendications 22 et 24, **caractérisé en ce que**
par l'exécution de l'étape f), l'objet (1,2,3,4,5) est recueilli dans une autre des percée (B2), de sorte que l'objet (1,2,3,4,5) se trouve à l'intérieur de cette percée (B2) lors d'une nouvelle exécution de l'étape b).

26. Procédé selon l'une des revendications 18 à 25, **caractérisé en ce que**
le dispositif de déplacement (S) présente une arête de poussée ou surface de poussée (SF) au moyen de laquelle l'objet (1,2,3,4,5) est poussé lors du pivotement en avant du dispositif de déplacement (S), le dispositif de déplacement (S) utilisé étant un de ceux dont l'arête de poussée ou la surface de poussée (SF) n'ont pas de tracé radial par rapport à l'axe de pivotement, de sorte que lors du déplacement de l'objet (1,2,3,4,5) par l'arête de poussée ou la surface de poussée, une composante de force due au frottement est exercée sur l'objet (1,2,3,4,5) vers l'intérieur en direction de l'axe de pivotement (A) ou vers l'extérieur, dans une direction opposée à l'axe de pivotement (A).

27. Procédé selon l'une des revendications 18 à 26, **caractérisé en ce que**
le support (U) utilisé est un de ceux qui sont partiellement formés par une table (T) sur laquelle l'objet (1,2,3,4,5) est poussé au moyen du dispositif de déplacement (S), la table (T) se déplaçant par rapport à une butée (ANS) ou par rapport à une racle (AS) de façon linéaire, de manière à ce que l'objet (1,2,3,4,5) poussé sur la table (T) se positionne du fait du frottement dans une position définie et au cas où l'objet (1,2,3,4,5) présente une forme oblongue, s'oriente dans un sens défini.

28. Procédé selon l'une des revendications 18 à 27, **caractérisé en ce que**
- le support (U) utilisé est formé partiellement par une table (T) sur laquelle l'objet (1,2,3,4,5) ou ses débris sont poussés au moyen du dispositif de déplacement (S) et au-dessous de laquelle se trouve un espace vide (H), et
- une racle (AS) disposée au-dessus de la table (T) est utilisée, la table (T) se déplaçant horizontalement par rapport à la racle (AS), de manière à ce que l'objet (1,2,3,4,5) ou ses débris sont raclés de la table (T) et tombent dans l'espace vide (H).

29. Procédé selon l'une des revendications 26 à 27, ainsi que selon la revendication 28, **caractérisé en ce que**
la surface de poussée (SF) est utilisée comme racle (AS).

30. Procédé selon l'une des revendications 18 à 29, **caractérisé en ce que**
le dispositif de déplacement (S) peut, après l'exécution de l'étape b) et avant l'exécution de l'étape d) ou avant l'exécution de l'étape e), d'abord pivoter en avant d'un angle de pivotement en avant supplémentaire défini dϕ et pivoter ensuite en arrière d'un même angle de pivotement en arrière supplémentaire - dϕ opposé à l'angle de pivotement en avant supplémentaire dϕ.

31. Procédé selon l'une des revendications 18 à 30, **caractérisé en ce que**
au lieu des étapes e) et f), l'étape suivante est exécutée : le dispositif de déplacement (S) est soumis moyennant l'entraînement (SA) à un même mouvement de retour non contraire au mouvement de déplacement, mouvement par lequel le dispositif de déplacement (S) est ramené dans la position de départ sans déplacer ni toucher l'objet (1).
